# EUROPEAN PATENT APPLICATION

(11) **EP 3 632 208 A1**
(43) Date of publication of application: **08.04.2020**
(21) Application number: 19209890.3
(22) Date of filing: 13.06.2014
(51) Int. Cl.: A01N 1/02, A61K 31/17, A61K 31/4706

(54) **CELL STABILIZATION**

(30) Priority: 13.06.2013 US 201361834517 P
(62) Divisional of application: 14819510.0
(71) Applicant: Biomatrica, Inc., San Diego, CA 92121 (US)
(72) Inventor: MULLER-COHN, Judy, San Diego, CA 92121 (US); DIAZ, Paul, San Diego, CA 92121 (US); MULLER, Rolf, San Diego, CA 92121 (US); LIBERAL, Vasco, San Diego, CA 92121 (US); PEREZ-LADAGA, Albert, San Diego, CA 92121 (US)
(74) Representative: Glawe, Delfs, Moll

(57) **Abstract**

The present invention relates to stabilization of cells at ambient temperatures. More particularly, the present invention relates to formulations, compositions, kits and methods that allow dehydration and rehydration of cells and dramatically increased recovery of functional cells after dry storage at room temperature.

## Description

### BACKGROUND OF THE INVENTION

This application claims the benefit of US Provisional Application No. 61/834,517, filed June 13, 2013, which is incorporated herein by reference in its entirety.

### TECHNICAL FIELD

The present invention relates to stabilization of cells.

### DESCRIPTION OF RELATED ART

The long-term storage of nucleated cells, e.g., eukaryotic cells, usually requires ultra-cold temperatures in the presence of the toxic cryoprotectant DMSO. In addition to losses caused by common failures of the cold storage systems, recovery of viable cells from the frozen state is challenging and typically only a small fraction of the input cells survive.

During the past 20 years, millions of dollars in research funds have been spent in trying to develop alternative methods for stabilization of eukaryotic cells at ambient temperatures. The driving force behind these efforts is the advantage of ambient temperature stabilized eukaryotic cell stocks for biomedical research, product development and direct applications in diagnostics, regenerative medicine, therapeutics, blood supply logistics and cell transplantation.

Based on the studies of extremophiles such as Tardigrades, rotifers or brine shrimp that can survive for many years in the dry state it was hypothesized that techniques and protocols could be developed that mimic this molecular phenomena in eukaryotic cell cultures. Unfortunately even after decades of research, practical dry storage stabilization of cells was not achieved. The best described stabilization technology uses trehalose, a non-reducing sugar molecule, as a drying medium. Mammalian cells loaded with trehalose can be dried but require almost immediate rehydration, and even then survival is limited. It has been shown that storage of the dried cells for even few minutes results in complete cell death.

Thus, there is a need to develop formulations, compositions and methods that allow for substantially dry storage of cells at ambient temperatures that remain viable upon rehydration.

### SUMMARY OF THE INVENTION

The formulations, compositions and methods described herein advantageously maintain cells in a viable state when stored under substantially dry conditions such that upon rehydration the cells retain at least one functional property, e.g., cell viability, after dry storage for a period of at least one hour, and certain embodiments, at least a week significantly increasing the time available for shipping and storing viable cells without the need of refrigeration or lyophilization. In one aspect of the invention, compositions are provided comprising a cell substantially dry stored without refrigeration or optionally without lyophilization wherein upon rehydration of the cell after substantially dry storage for at least 1 hour the rehydrated cell exhibits at least one functional property that is substantially the same in the cell prior to dehydration and substantially dry storage.

In certain embodiments, the compositions comprise at least one dry storage stabilizer, preferably selected from the group consisting of amino acids, synthetic amino acids, peptides, non-reducing sugars, chelating agents, water-soluble polymers and tetrahydropyrimidines. In one embodiment, the no stabilizer that is a sugar molecule (e.g.; trehalose) is present, or if a stabilizer that is a sugar molecule is present, then another stabilizer that is not a sugar molecule is also present.

In certain other embodiments, compositions comprising at least one apoptosis inhibitor, preferably a reversible apoptosis inhibitor, are provided. Exemplary apoptosis inhibitors are selected from the group consisting of a PERK-eIF2-α inhibitor, an ASK1 inhibitor, a NRF2-KEAP1 inhibitor, a JNK inhibitor, a p38 MAP kinase inhibitor, an IRE1 inhibitor, a GSK3 inhibitor, a MEK inhibitor, a PI3K pathway inhibitor, a calpain inhibitor, and a caspase-1 inhibitor.

In another aspect, compositions are provided wherein prior to dehydration the cell is treated with a predehydration formulation comprising at least one apoptosis inhibitor to generate a pretreated cell. In certain embodiments, the least one apoptosis inhibitor is selected from the group consisting of wherein the apoptosis inhibitor is selected from the group consisting of a PERK-eIF2-α inhibitor, an ASK1 inhibitor, a NRF2-KEAP1 inhibitor, a JNK inhibitor, a p38 MAP kinase inhibitor, an IRE1 inhibitor, a GSK3 inhibitor, a PIK3 pathway inhibitor, a MEK inhibitor, a calpain inhibitor, and a caspase-1 inhibitor.

In one embodiment, the least one apoptosis inhibitor is a PERK-eIF2-α inhibitor.

In another embodiment, the least one apoptosis inhibitor is an ASK1 inhibitor.

In yet another embodiment, the least one apoptosis inhibitor is a NRF2-KEAP1 inhibitor.

In still another embodiment, the least one apoptosis inhibitor is a GSK3 inhibitor.

In one embodiment, the least one apoptosis inhibitor is a MEK inhibitor.

In another embodiment, the least one apoptosis inhibitor is a JNK inhibitor.

In yet another embodiment, the least one apoptosis inhibitor is a JNK inhibitor and a p38 MAP kinase inhibitor.

In still another embodiment, the least one apoptosis inhibitor is a PI3K inhibitor.

In one embodiment, the least one apoptosis inhibitor is an IRE-1 inhibitor.

In another embodiment, the least one apoptosis inhibitor is a calpain inhibitor.

In yet another embodiment, the least one apoptosis inhibitor is a casapase-1 inhibitor.

In certain embodiments, the predehydration formulation comprises at least one apoptosis inhibitor and at least one ER chaperone inducer.

In certain other embodiments, the predehydration formulation comprises at least one apoptosis inhibitor and at least one autophagy inducer.

In yet another embodiment, the predehydration formulation comprises at least one apoptosis inhibitor and at least one survival protein.

In another aspect of the invention, compositions are provided comprising a rehydrated cell, wherein the rehydrated cell is a cell substantially dry stored without refrigeration or lyophilization wherein upon rehydration of the cell after substantially dry storage for at least 1 hour the rehydrated cell exhibits at least one functional property that is substantially the same in the cell prior to dehydration and substantially dry storage.

In certain embodiments, the rehydration of the cell occurs in the presence of a rehydration formulation, and preferably the rehydration formulation comprises at least one apoptosis inhibitor.

In certain other embodiments, the at least one apoptosis inhibitor in the rehydration buffer is selected from the group consisting of a PERK-eIF2-α inhibitor, an ASK1 inhibitor, a NRF2-KEAP1 inhibitor, a JNK inhibitor, a p38 MAP kinase inhibitor, an IRE1 inhibitor, a GSK3 inhibitor, a MEK inhibitor, a PI3K pathway inhibitor, a calpain inhibitor, and a caspase-1 inhibitor.

In yet other embodiments, the rehydration formulation comprises an apoptosis inhibitor one or more of the following selected from the group consisting of an ER chaperone inducer, an autophagy inducer and a survival protein.

In one embodiment, the at least one functional property after rehydration comprises metabolic activity.

In certain embodiments, the metabolic activity is measured by determining ATP content or a caspase determination assay.

In certain other embodiments, the metabolic activity after rehydration is measured 24 hours after rehydrating the cell, 48 hours after rehydrating the cell, 72 hours after rehydrating the cell, or one week after rehydrating the cell.

In other embodiments, the composition is stabilized in dehydrated form for at least 24 hours prior to rehydration, for at least 48 hours prior to rehydration, for at least 72 hours prior to rehydration or for at least one week prior to rehydration.

In other aspect, dehydration formulations are provided comprising a pH buffer; a synthetic amino acid, a water-soluble polymer; and a first amino acid or a peptide. In certain embodiments, the dehydration formulation further comprising a non-reducing sugar, at least one apoptosis inhibitor or a second amino acid.

In still another aspect, methods are provided for substantially dry storage of one or more cell at ambient temperatures in the absence of lypholization, comprising incubating one or more cell in a dehydration formulation and dehydrating the one or more pretreated cell in the presence of a dehydration formulation to generate one or more substantially dry stored cell. In still a further methods are provided for substantially dry storage of one or more cell at ambient temperatures in the absence of lypholization, comprising incubating the one or more cell with a predehydration formulation comprising an apoptosis inhibitor to generate one or more pretreated cell, removing the predehydration formulation; and dehydrating the one or more pretreated cell in the presence of a dehydration formulation to generate one or more substantially dry stored cell.

In one embodiment, the dehydration formulation used in the method comprises at least one dry storage stabilizer and may further comprise at least one apoptosis inhibitor, in one embodiment a reversible apoptosis inhibitor, when a predehydration formulation is not used.

In another embodiment, the method further comprises immobilizing one or more cell to a solid support prior to incubating the one or more cell with the predehydration formulation.

In yet another embodiment, the method further comprises rehydrating the one or more substantially dry stored cell to generate a rehydrated cell using a rehydration formulation comprising at least one apoptosis inhibitor.

In certain embodiments, the at least one apoptosis inhibitor in the predehydration formulation is the same as the at least one apoptosis inhibitor in the rehydration formulation, and in other embodiments the at least one apoptosis inhibitor in the predehydration formulation is different from the at least one apoptosis inhibitor in the rehydration formulation.

### BRIEF DESCRIPTION OF THE DRAWINGS

Figure 1 is a schematic representation of the three proximal ER transmembrane sensors of the ER stress pathway (A) GRP78-bound inactivated state and (B) release of GRP78 results in activation IRE1.
Figure 2 is a schematic representation of the ER Stress Pathway (A) (B) blocked steps and targets of the ER stress pathway inhibited by apoptosis inhibitors.
Figure 3 shows that mesenchymal stem cells (MSCs) substantially dry stored using the formulations and methods described herein retain the ability upon rehydration to differentiate into adipocytes (Panel B), osteocytes (Panel C), and chondrocytes (Panel D).
Figure 4 illustrate long term viability of HeLa cells after dehydration and 5 hours storage at room temperature. **(A)** Cell viability after 5 hours dry followed by five days of rehydration. **(B)** Number of living cells per ml. Cells were seeded into fresh cell culture plates, and the groups treated with composition according to one embodiment disclosed herein.

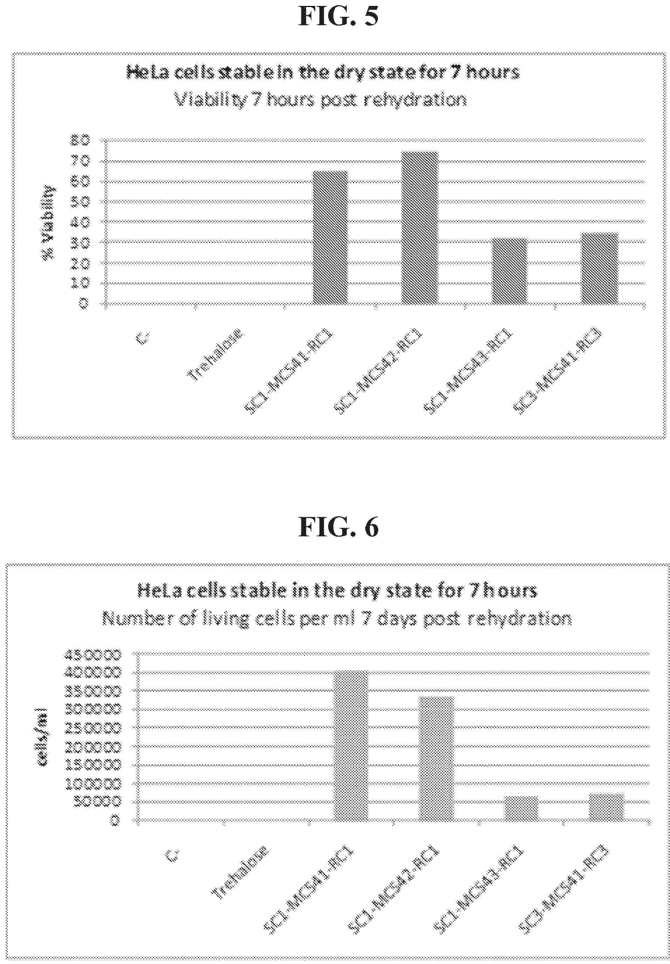

Figure 5 shows Caspase 3/7 activation relative to non-treated controls. Caspase activation was calculated as a ratio of activity in the test samples relative to untreated cells, cultured under standard tissue culture conditions. Test cells were dehydrated and stored at room temperature dry for 5 hours, followed by 5 days of rehydration.
Figure 6 shows survival of cells after dehydration, 7 hours of storage in the dry state and rehydration after storage. Cell Viability was evaluated 3 days after reseeding and a total of 7 days after rehydration. Different stabilization formulations impact the cell survival as well as cell proliferation. Unprotected control and trehalose stabilized cells did not yield in any surviving cells.

### DETAILED DESCRIPTION OF THE INVENTION

Without wishing to be bound by any theory, it is contemplated that the formulations and compositions of certain embodiments described herein stabilize the integrity of cellular membranes and organelles of cells while also blocking apoptosis, e.g., by blocking specific ER stress pathways at defined stages prior to dehydration and at the time of rehydration, to provide substantially dry cells stored at ambient temperatures for at least one hour that retain at least one functional property after being rehydrated for a period of at least one hour. In certain embodiments, the specific ER stress pathways are blocked using an apoptosis inhibitor. In other embodiments, the specific ER stress pathways are blocked using an apoptosis inhibitor in combination with an ER chaperone inducer to drive the ER stress pathway towards an adaptation response rather than apoptosis.

In certain embodiments, the cells are dehydrated using a dehydration formulation, preferably comprising at least one dry storage stabilizer and at least one apoptosis inhibitor.

In certain other embodiments, cells are pretreated using a predehydration formulation comprising an apoptosis inhibitor for a predetermined period of time, e.g., 1 hr, prior to dehydrating the cells in the presence of a dehydration formulation to produce substantially dry stored cells. The substantially dry stored cells are rehydrated in the presence of a rehydration formulation, preferably comprising an apoptosis inhibitor, which may be the same or different than the apoptosis inhibitor in the predehydration formulation. The rehydration formulation is removed after a specified period of time, e.g., 1 hr, and the cells may be rehydrated in growth medium or other suitable solutions and buffers depending on the intended end use.

### DEFINITIONS

Unless defined otherwise, all technical and scientific terms used herein have the same meaning as is commonly understood by one of skill in the art to which this invention belongs. All patents, patent applications and publications referred to herein are incorporated by reference in their entirety.

As used herein, the term "eukaryotic cell" refers to at least one nucleated cell present in a body fluid or tissue of a eukaryotic organism, preferably a human, at any given stage of development of the eukaryote, including fertilized oocytes, blast cells and other embryonic stages, fetus or adult. Exemplary cells that may be substantially dry stored at ambient temperatures using the formulations, compositions and methods of the present invention include, but are not limited to, fibroblasts, keratinocytes, chondrocytes, melanocytes, osteoblasts, osteocytes, myocytes, cardiomyocytes, neurons, Schwann cells, glial cells, astrocytes, oligodendrocytes, T-cells, B-cells, memory cells, reticulocytes, monocytes, neutrophils, basophils, eosinophils, macrophages, megakaryocytes, dendritic cells, adipocytes, islet cells, oocytes, spermatocytes, placental cord blood cells, blast cells, zygotes, epithelial cells (e.g., mammary gland cells, endometrial cells, pancreatic acinar cells, goblet cells, Langerhans cells, ameloblasts and paneth cells), odontocytes, hepatocytes, lipocytes, parietal cells, pneumocytes, endothelial cells, tumor cells, circulating tumor cells, retinal photoreceptor and pigment cells, lens cells, and stem cells, including pluripotent or totipotent embryonic, fetal, iPS cells, and mesenchymal, or mixtures thereof. The stem cells may be substantially dry stored at ambient temperatures in an undifferentiated or partially differentiated state.

As used herein, the term "pretreated cell" refers to a cell that has been treated with a predehydration formulation comprising at least one apoptosis inhibitor prior to dehydration. The pretreated cell is preloaded with the apoptosis inhibitor to allow for substantially dry storage of the pretreated cell without ER stress pathway activation using dehydration formulations containing or lacking an apoptosis inhibitor. In certain embodiments, the predehydration formulation comprising the apoptosis inhibitor is removed from the pretreated cells prior to adding the dehydration formulation, but the intracellular concentration of the apoptosis inhibitor is sufficient to substantially dry store the cell without ER stress pathway activation.

As used herein, the term "substantially dry storage at ambient temperatures" or "substantially dry stored at ambient temperatures" refers to the ability to store cells at ambient temperatures while maintaining at least one functional property of the cell in a re-hydrated state without refrigeration or lyophilization using the formulations, compositions and methods of the present invention. The substantially dry stored cells do not necessarily need to be devoid of all free internal water, but preferably at least 45%, at least 50%, at least 60%, at least 70%, at least 80%, at least 90%, at least 95% or up to 98% of the free internal water is removed. The substantially dry stored cell may be stored at ambient temperatures for various periods of time depending on the type of cell to be dry stored, the predehydration and/or dehydration formulation used and the intended use for the substantially dry stored cell. The cell may be stored in a dehydrated state for a period of: 1) hours, e.g., one, two, six, twelve or eighteen hours; 2) days, e.g., one day, two days, four days or six days; 3) weeks, e.g., one week, two weeks or three weeks; 4) months, e.g., one month, two months, four months, six months, eight months or eleven months; and 5) even years, e.g., one year, two years, five years, ten years, twenty years or more.

As used herein, the term "immobilized" refers to substantially dry stored cells that are adhered to or are in direct contact with a solid surface. The cells may be immobilized prior to the addition of the dehydration formulation and drying or maybe immobilized prior to the predehydration step and maintained immobilized throughout the dehydration process. Suitable solid surfaces include, but are not limited to, glass slides, beads, chips, membranes, sheets, meshes, columns, affinity resins, sponges, plastic, including 96 well plates, culture dishes and flasks, tubes, containers, vessels, natural matrices such as but not limited to collagen and alginate hydrogels, or any other substratum whereby cells may be grown..

As used herein, an "apoptosis inhibitor" refers to any compound or agent capable of downregulating, decreasing, suppressing or otherwise regulating the amount and/or activity of a desired enzyme or pathway, preferably a step in an ER stress pathway to prevent induction of cellular apoptosis, including ER chaperone inducers, autophagy inducers and survival protein endogenous or exogenous. Inhibition of these enzymes or pathways can be achieved by any of a variety of mechanisms known in the art, including, but not limited to binding directly to the enzyme, preferably in a reversible manner, or transiently inhibiting the expression of the gene (e.g., transcription to mRNA, translation to a nascent polypeptide, and/or final polypeptide modifications to a mature protein), which encodes the enzyme or target. An apoptosis inhibitor includes the specific apoptosis inhibitors described herein.

As used herein the term "inhibiting" or "inhibition" refers to the ability of an compound or agent to downregulate, decrease, reduce, suppress, inactivate, or inhibit at least partially the activity of an enzyme, or the expression of an enzyme or protein. Preferably, the inhibition is reversible.

### ER STRESS PATHWAY

In certain embodiments, the predehydration formulation, the dehydration formulation and/or rehydration formulation comprises at least one apoptosis inhibitor that blocks at least one essential step in the ER stress pathway.

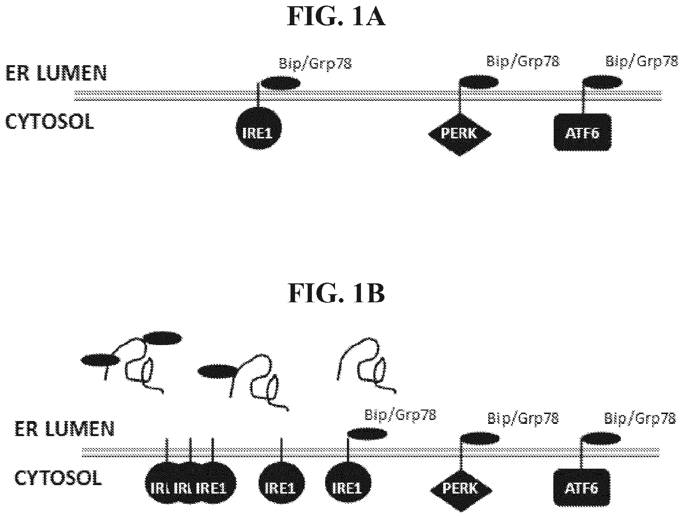

As shown in Fig. 1A, unfolded protein response (UPR) signaling in higher eukaryotes is initiated by three proximal ER transmembrane sensors, PERK, the kinase/RNase IRE1, and the transcription factor ATF6 (e.g., see Kaufman RJ (2002) J Clin Invest 110: 1389-1398. doi: 10.1172/jci0216886; Mori K (2000) Cell 101: 451-454. doi: 10.1016/s0092-8674(00)80855-7; Ron D, Walter P (2007) Nat Rev Mol Cell Biol 8: 519-529. doi: 10.1038/nrm219).The activation of PERK leads to inhibition of protein translation on a global scale by phosphorylation of eIF2α, α translation initiation factor (Fig 1B; Harding et al., (2000) Mol Cell 5: 897-904. doi: 10.1016/s1097-2765(00)80330-5). Concomitantly, PERK promotes transcription of UPR-specific genes by increasing translation of the transcription factor ATF4. IRE1 generates an alternatively spliced and more potent form of XBP1 by excises an intron from XBP1 mRNA (e.g., see Calfon et al. (2002) Nature 415: 92-96. doi: 10.1038/415092a). The third UPR sensor, ATF6, is an ER transmembrane protein with a transcription activation domain on its cytoplasmic side.

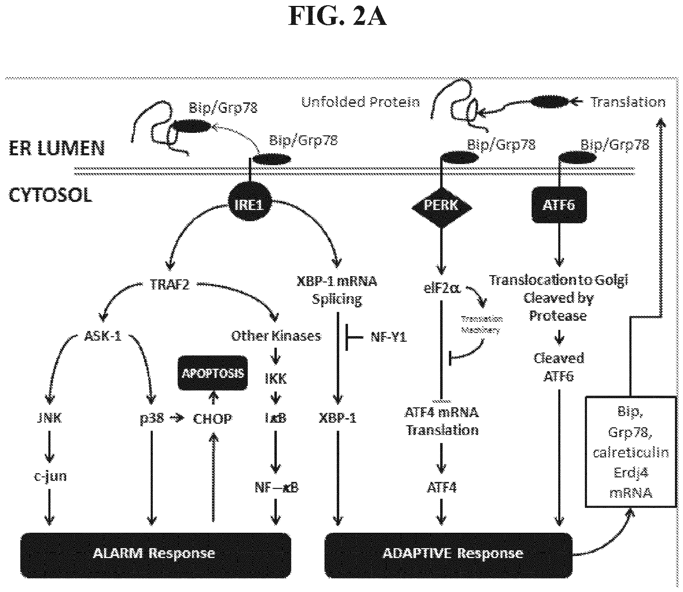

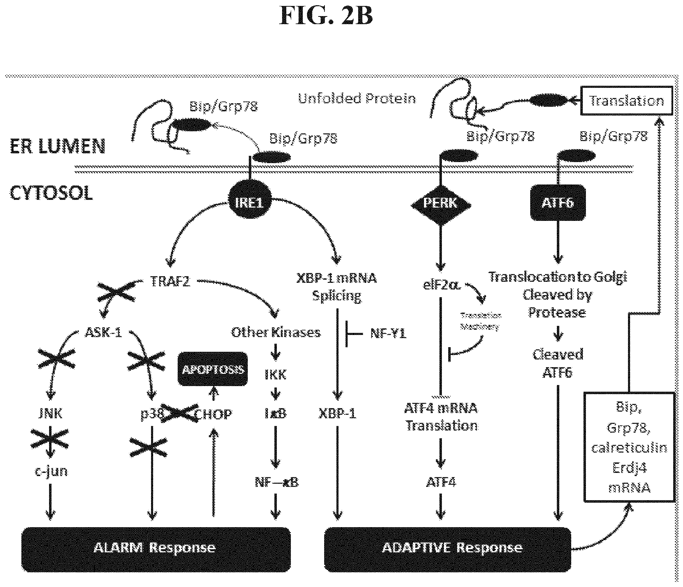

As shown in Fig 2A, during ER stress events, ATF6 undergoes proteolysis thus liberating its cytoplasmic transactivation domain from the ER membrane. Once free it enters the nucleus (Haze et al., (1999) Mol Biol Cell 10: 3787-3799. doi: 10.1091/mbc.10.11.3787) and initiates transcription of additional UPR gene. The activation of the proximal sensors of ER stress by the UPR result in a complex pattern of gene regulation. Thus the UPR signals aim to alleviate and reduce the high levels of misfolded proteins in the ER by increasing protein folding capacity through up-regulation of ER chaperones such as BiP, GRP94, calreticulin, and Erdj4 (e.g., see Okada et al., (2002) Biochem J 366: 585-594. doi: 10.1042/bj20020391; Yoshida et al., (1998) J Biol Chem 273: 33741-33749. doi: 10.1074/jbc.273.50.33741). In the event, however, that proper protein folding in the ER cannot be restored, genes such as CHOP are upregulated and can result in the activation of apoptotic pathways.

### 1. GRP78/BiP

GRP78/BiP a member of the HSP family of molecular chaperones required for endoplasmic reticulum integrity and stress-induced autophagy. GRP78 plays a central role in regulating the unfolded protein response (UPR), and is an obligatory component of autophagy in eukaryotic cells and may play an important role in cellular adaptation and oncogenic survival. One of the client proteins of GRP78 is protein double-stranded RNA-activated protein-like endoplasmic reticulum kinase (PERK), and binding to PERK precludes PERK oligomerization. GRP78 also binds to client proteins IRE1 and ATF6 to prevent oligomerization of IRE1 and activation of ATF6. GRP78 plays a role in facilitating the assembly of multimeric protein complexes inside the ER.

### 2. IRE1

Inositol-requiring enzyme 1 (IRE1) a ser/thr protein kinase that possess endonuclease activity. IRE1 is important in altering gene expression as a response to endoplasmic reticulum based stress signals and senses unfolded proteins in the lumen of the endoplasmic reticulum via its N-terminal domain, which leads to enzyme auto-activation. The active endoribonuclease domain splices XBP1 mRNA to generate a new C-terminus, converting it into a potent unfolded-protein response transcriptional activator and triggering growth arrest and apoptosis. The kinase domain is activated by trans-autophosphorylation and the kinase activity is required for activation of the endoribonuclease domain. IRE1 is ubiquitously expressed and high levels are observed in pancreatic tissue. IRE1 is a disulfide-linked homodimer and dimer formation is driven by hydrophobic interactions within the N-terminal luminal domains and stabilized by disulfide bridges. IRE1 also binds HSPA5, a negative regulator of the unfolded protein response. This interaction may disrupt homodimerization and prevent activation of IRE1.

### 3. PERK

Eukaryotic translation initiation factor 2-alpha kinase 3, also known as PRKR-like endoplasmic reticulum kinase or protein kinase R (PKR)-like endoplasmic reticulum kinase (PERK), is an enzyme that in humans is encoded by the *EIF2AK3* gene. PERK phosphorylates the alpha subunit of eukaryotic translation-initiation factor 2 (EIF2), leading to its inactivation, and thus to a rapid reduction of translational initiation and repression of global protein synthesis. It is a type I membrane protein located in the endoplasmic reticulum (ER), where it is induced by ER stress caused by malfolded proteins.

### 4. ATF6

This gene encodes a transcription factor that activates target genes for the unfolded protein response (UPR) during endoplasmic reticulum (ER) stress. Although it is a transcription factor, this protein is unusual in that it is synthesized as a transmembrane protein that is embedded in the ER. It functions as an ER stress sensor/transducer, and following ER stress-induced proteolysis, it functions as a nuclear transcription factor via a cis-acting ER stress response element (ERSE) that is present in the promoters of genes encoding ER chaperones. This protein has been identified as a survival factor for quiescent but not proliferative squamous carcinoma cells.

### 5. ASK1

Apoptosis signal-regulating kinase 1 (ASK1) also known as mitogen-activated protein kinase kinase kinase 5 (MAP3K5) is a member of MAP kinase kinase kinase family and as such a part of mitogen-activated protein kinase pathway. ASK1 directly phosphorylates MKK4 (SEK1)/MKK7 and MKK3/MKK6, which in turn activates c-Jun N-terminal kinase (JNK) and p38 mitogen-activated protein kinases in a Raf-independent fashion in response to an array of stresses such as oxidative stress, endoplasmic reticulum stress and calcium influx.

Under nonstress conditions ASK1 is oligomerized (a requirement for its activation) through its C-terminal coiled-coil domain (CCC), but remains in an inactive form by the suppressive effect of reduced thioredoxin (Trx) and calcium and integrin binding protein 1 (CIB1). Trx inhibits ASK1 kinase activity by direct binding to its N-terminal coiled-coil domain (NCC). Trx and CIB1 regulate ASK1 activation in a redox- or calcium- sensitive manner, respectively. Both appear to compete with TNF-α receptor-associated factor 2 (TRAF2), an ASK1 activator. TRAF2 and TRAF6 are then recruited to ASK1 to form a larger molecular mass complex (see Fig 2A). Subsequently, ASK1 forms homo-oligomeric interactions not only through the CCC, but also the NCC, which leads to full activation of ASK1 through autophosphorylation at threonine 845.

### 6. JNK

The c-Jun-N-terminal kinases (JNK1/2/3) are the downstream components of one of the three major groups of mitogen-activated protein kinase (MAPK) cascades found in mammalian cells, with the other two consisting of the extracellular signal-regulated kinases (ERK1/2) and the p38 protein kinases (p38.α, β, γ, ,δ). Each group of kinases is part of a three-module cascade that include a MAPK, which is activated by phosphorylation by a MAPK kinase (MAPKK), which in turn is activated by phosphorylation by a MAPKK kinase (MAPKKK). Activation of JNK and p38 have been linked to the induction of apoptosis. Using many cell types, it was shown that persistent activation of JNK induces cell death, and that the blockade of JNK activation by dominant-negative (DN) inhibitors prevents killing by an array of apoptotic stimuli. The role of JNK in apoptosis is also documented by the analyses of mice with targeted disruptions of jnk genes. Mouse embryonic fibroblasts (MEFs) lacking both JNK1 and JNK2 are completely resistant to apoptosis by various stress stimuli, including genotoxic agents, UV radiation, and anisomycin, and jnk3-/- neurons exhibit a severe defect in the apoptotic response to excitotoxins. Moreover, JNK2 was shown to be required for anti-CD3-induced apoptosis in immature thymocytes.

### 7. p38 MAP Kinases

p38 MAP kinases (α, β, γ, and δ) are members of the MAPK family and four p38 MAPKs have been cloned in higher eukaryotes: p38-Alpha/XMpk2/CSBP, p38-Beta/p38-Beta22, p38-Gamma/SAPK3/ERK6, and p38-Delta/SAPK4. These four proteins are 60-70% identical in their amino acid sequence and are all activated by MKK6 (MAPK Kinase-6). Another MAPK kinase, MKK3 (MAPK Kinase-3), has been shown to phosphorylate and activate p38-Alpha, p38-Gamma, and p38-Delta but not p38-Beta2. The mammalian p38 MAPK families are activated by cellular stress including UV irradiation, heat shock, and high osmotic stress.

The activation of p38 MAP kinase can also directly influence gene transcription, as a growing number of transcription factors are known to be direct targets of p38. Direct phosphorylation and activation have been described for ATF1, ATF2, and ATF-6, the MEF2A/C (Myocyte Enhance Factor-2A/C), SAP1A (Signaling lymphocytic Activation molecule associated Protein-1A) and the Elk1 (ETS-domain transcription factor-1).

### 8. MEK

"MEK1" and "MEK2," are abbreviations for mitogen-activated ERK-activating kinases (where ERK is extracellular signal-regulated protein kinase, another designation for MAPK). MEK1 and MEK2 are dual-function serine/threonine and tyrosine protein kinases and are also known as MAP kinases. Ras-GTP activates Raf, which activates MEK1 and MEK2, which activate MAP kinase (MAPK). Once activated, Raf and other kinases phosphorylate MEK on two neighboring serine residues, S²¹⁸ and S²²² in the case of MEK-1. These phosphorylations are required for activation of MEK as a kinase. In turn, MEK phosphorylates MAP kinase on two residues separated by a single amino acid: a tyrosine, Y¹⁸⁵, and a threonine, T¹⁸³. MEK appears to associate strongly with MAP kinase prior to phosphorylating it, suggesting that phosphorylation of MAP kinase by MEK may require a prior strong interaction between the two proteins.

### 9. PI3K-Akt Pathway

The phosphatidylinositol 3' -kinase (PI3K)-Akt signaling pathway is activated by many types of cellular stimuli or toxic insults and regulates fundamental cellular functions such as transcription, translation, proliferation, growth, and survival. The binding of growth factors to their receptor tyrosine kinase (RTK) or G protein-coupled receptors (GPCR) stimulates class Ia and Ib PI3K isoforms, respectively. PI3K catalyzes the production of phosphatidylinositol-3,4,5-triphosphate (PIP3) at the cell membrane. PIP3 in turn serves as a second messenger that helps to activate Akt. Once active, Akt can control key cellular processes by phosphorylating substrates involved in apoptosis, protein synthesis, metabolism, and cell cycle.

### 10. XBP-1

This gene encodes a transcription factor that regulates MHC class II genes by binding to a promoter element referred to as an X box. This gene product is a bZIP protein, which was also identified as a cellular transcription factor that binds to an enhancer in the promoter of the T cell leukemia virus type 1 promoter. It has been found that upon accumulation of unfolded proteins in the endoplasmic reticulum (ER), the mRNA of this gene is processed to an active form by an unconventional splicing mechanism that is mediated by the endonuclease inositol-requiring enzyme 1 (IRE1). The resulting loss of 26 nt from the spliced mRNA causes a frame-shift and an isoform XBP1(S), which is the functionally active transcription factor. The isoform encoded by the unspliced mRNA, XBP1(U), is constitutively expressed, and thought to function as a negative feedback regulator of XBP1(S), which shuts off transcription of target genes during the recovery phase of ER stress. A pseudogene of XBP1 has been identified and localized to chromosome 5.

### 11. eIF2-α

eIF2-alpha a translation initiation factor that functions in the early steps of protein synthesis by forming a ternary complex with GTP and initiator tRNA. This complex binds to a 40s ribosomal subunit, followed by mRNA binding to form a 43S preinitiation complex. Junction of the 60S ribosomal subunit to form the 80S initiation complex is preceded by hydrolysis of the GTP bound to eIF-2 and release of an eIF-2-GDP binary complex. In order for eIF-2 to recycle and catalyze another round of initiation, the GDP bound to eIF-2 must exchange with GTP by way of a reaction catalyzed by eIF-2B. eIF2-alpha is phosphorylated by at least 4 kinases: PERK, GCN2, HRI and PKR, and phosphorylation stabilizes the eIF-2/GDP/eIF-2B complex and prevents GDP/GTP exchange reaction, thus impairing the recycling of eIF-2 between successive rounds of initiation and leading to global inhibition of translation.

### 12. GSK3

Glycogen synthase kinase-3 (GSK3) was initially identified as an enzyme involved in the control of glycogen metabolism. In recent years it has been shown to have key roles in regulating a diverse range of cellular functions, including initiation of protein synthesis, cell proliferation, cell differentiation, apoptosis, and is essential for embryonic development as a component of the Wnt signaling cascade. GSK3 as a central negative regulator in the insulin signaling pathway and plays a role in insulin resistance.

### 13. NRF2

Nuclear factor (erythroid-derived 2)-like 2, also known as NFE2L2 or NRF2, is a transcription factor that in humans is encoded by the *NFE2L2* gene. The NRF2 antioxidant response pathway is the primary cellular defense against the cytotoxic effects of oxidative stress. NRF2 increases the expression of several antioxidant enzymes.

NRF2 is a basic leucine zipper (bZIP) transcription factor. Under normal or unstressed conditions, NRF2 is kept in the cytoplasm by Kelch like-ECH-associated protein 1 (KEAP1) and Cullin 3 which degrade NRF2 by ubiquitination. Under oxidative stress, NRF2 is not degraded, but instead travels to the nucleus where it initiates transcription of antioxidative genes and their proteins.

### 14. KEAP1

Kelch-like ECH-associated protein 1 (KEAP1) has been shown to interact with NRF2, a master regulator of the antioxidant response. Under quiescent conditions, NRF2 is anchored in the cytoplasm through binding to KEAP1, which, in turn, facilitates the ubiquitination and subsequent proteolysis of NRF2. Such sequestration and further degradation of NRF2 in the cytoplasm are mechanisms for the repressive effects of KEAP1 on NRF2.

### 15. ATF4

Activating transcription factor 4 (tax-responsive enhancer element B67), also known as ATF4, is a protein that in humans is encoded by the *ATF4* gene. This gene encodes a transcription factor that was originally identified as a widely expressed mammalian DNA binding protein that could bind a tax-responsive enhancer element in the LTR of HTLV-1. The encoded protein was also isolated and characterized as the cAMP-response element binding protein 2 (CREB-2). The protein encoded by this gene belongs to a family of DNA-binding proteins that includes the AP-1 family of transcription factors, cAMP-response element binding proteins (CREBs) and CREB-like proteins. These transcription factors share a leucine zipper region that is involved in protein-protein interactions, located C-terminal to a stretch of basic amino acids that functions as a DNA-binding domain. Two alternative transcripts encoding the same protein have been described. Two pseudogenes are located on the X chromosome at q28 in a region containing a large inverted duplication.

### DRY STORAGE STABILZERS

### 1. Natural and Synthetic Amino Acids

Also as described herein, certain embodiments the dehydration formulation may include at least one amino acid or synthetic amino acid in the dehydration formulation for substantially dry storage of functional cells at ambient temperatures.

In certain embodiments, the natural amino acid is selected from the group consisting of glycine, glutamine, glutamic acid, and proline.

In certain other embodiments, the dehydration formulation and compositions may contain one or more synthetic amino acid having a general formula I 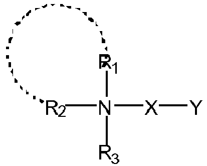 wherein R₁, R₂, R₃ are independently selected from aryl, arylalkyl, -H, -CH₃ and -CH₂-CH₃, wherein when R₁ and R₂ are CH₃ or CH₂-CH₃, R₃ is either H or absent, wherein X is selected from -CH₂-, -CH₂CH₂-, -CH₂CH₂CH₂-, -CH₂CH₂CH₂CH₂-, 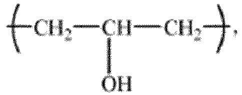 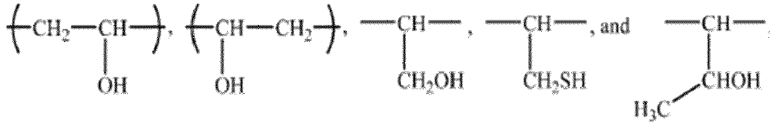 and wherein Y is selected from COO and SO3

Exemplary synthetic amino acids useful in the compositions include those described in WO2010132508 and US Patent 8,519,125, the content of which are incorporated herein by reference in their entirety. Synthetic amino acids that can be employed with the present compositions include hydroxyproline and betaine (N,N,N-trimethylglycine).

### 2. Peptides

The herein described dehydration formulations for substantially dry storage of cells at ambient temperatures may, in certain embodiments, contain a peptide. Advantageously, it has been determined that certain dehydration formulations, including those set forth in Table 1, comprising a stabilizing amount of a small dipeptide or dipeptide analog, e.g., between about 10 mM and 200 mM, are unexpectedly capable of substantially dry storage of cells at ambient temperatures and for period of time that exceed cold storage of these cells. An examplary dipeptide has the amino acid sequence alanine - glutamine.

### 3. Trisaccharides

As described herein, certain embodiments described herein the formulations may include at least one trisaccharide in the dehydration formulation or composition for substantially dry storage of a cell at ambient temperatures. Trisaccharides are oligosaccharides composed of three monosaccharides with two glycosidic bonds connecting them. The glycosidic bond can be formed between any hydroxyl group on the component monosaccharides and different bond combinations (regiochemistry) and stereochemistry (alpha- or beta-) result in triaccharides that are diastereoisomers with different chemical and physical properties. Selection of one or more particular trisaccharide for inclusion in a stable storage composition may be done based on the present disclosure and according to routine practices in the art, and may be influenced by a variety of factors including other formulation components. Exemplary trisaccharides include, but are not limited to, maltotrose, isomaltotriose, raffinose, melezitriose, nigerotriose and ketose. In certain embodiments for substantially dry storage of cells, including formulations set forth in Table 1, the trisaccharide is melezittriose and preferably at a concentration of about 1% - 20%, even more preferably about 5.0 - 15 %, where "about" may be understood to represent quantitative variation that may be more or less than the recited amount by less than 25%, more preferably less than 20%, and more preferably less than 15%, 10%, 5% or 1%.

### 4. Water-soluble Polymers

As described herein, certain embodiments may include at least one water-soluble polymer in the formulations and compositions for substantially stable storage of nucleic acid and/or polypeptide molecules in a whole blood sample. Such water soluble polymers include polyvinyl pyrrolidine and polyvinyl alcohol and it will be appreciated that from the present disclosure the skilled person may select other water soluble polymers for use in a substantially dry storage formulations and compositions, as may vary based on the other components of the composition that are employed and the particular cell type being stored. Certain embodiments, including but not limited to those presented in Table 1, contemplate inclusion of a water-soluble polymer at a concentration (on a volumetric basis, i.e., vol/vol) of about 0.1 to 10% (vol/vol), more preferably between of about 0.1 to 5% (vol/vol), and even more preferably 1.0% (vol/vol)where "about" may be understood to represent quantitative variation that may be more or less than the recited amount by less than 50%, more preferably less than 40%, more preferably less than 30%, and more preferably less than 20%, 15%, 10% or 5%. In certain embodiments, the water-soluble polymer is polyvinyl alcohol with a molecular weight range of about 30-70,000 daltons and about 87-90% hydrolyzed, where "about" may be understood to represent quantitative variation that may be more or less than the recited amount by less than 50%, more preferably less than 40%, more preferably less than 30%, and more preferably less than 20%, 15%, 10% or 5%.

### 5. Non-reducing Sugars

Also as described herein, certain embodiments may include at least one non-reducing sugar in the predehydration and/or dehydration formulations and compositons at ambient temperatures. Non-reducing sugars are carbohydrate molecules that lack a functional aldehyde group. Exemplary non-reducing sugars include sucrose and trehalose. In embodiments for substantially dry storage of cells, the non-reducing sugar is trehalose present at a concentration of about 1.0 - 200 mM, preferably about 50 mM - 200 mM, and more preferably about 150 mM, where "about" may be understood to represent quantitative variation that may be more or less than the recited amount by less than 25%, more preferably less than 20%, and more preferably less than 15%, 10%, 5% or 1%.

### 6. Polyethers

Polyethers may, according to certain embodiments, be included in the presently described dehydration formulations for substantially stable storage of functional cells at ambient temperatures. Polyether generally refers to polymers which contain the more than one ether functional group in their main chain. Polyethers are relatively stable compounds formed by the dehydration of alcohols. Exemplary polyethers for use in the formulations, compositions and methods include, but are not limited to, polyethylene glycol, polypropylene glycol, and polyphenyl ethers. In certain embodiments, the molecular weight of the polyether is between about 5,000 and 15,000 daltons.

### 7. Tetrahydropyrimidines

In certain embodiments, the dry storage stabilizer is a tetrhydropyrimidine. An exemplary tetrahydropyrmidine is 5-hydroxyectoine. In certain dehydration formulations and compositions 5-hydroxyectoine is used at a concentration between about 10 mM and about 200 mM.

### FORMULATION REAGENTS

### 1. pH Buffers

According to certain embodiments the herein described dehydration formulations and compositions for substantially dry storage of a functional cell at ambient temperatures may include one or more pH buffer, which may be any of a large number of compounds known in the art for their ability to resist changes in the pH of a solution, such as an aqueous solution in which the pH buffer is present. Selection of one or more particular pH buffers for inclusion in a stable storage composition may be done based on the present disclosure and according to routine practices in the art, and may be influenced by a variety of factors including the pH that is desirably to be maintained, the nature of the biological sample, the solvent conditions to be employed, the other components of the formulation to be used, and other criteria. For example, typically a pH buffer is employed at a pH that is within about 0.1, 0.2, 0.3, 0.4, 0.5, 0.6, 0.7, 0.8, 0.9 or 1.0 pH unit of a proton dissociation constant (pKₐ) that is a characteristic of the buffer.

Non-limiting examples of pH buffers include citric acid, tartaric acid, malic acid, sulfosalicylic acid, sulfoisophtalic acid, oxalic acid, borate, CAPS (3-(cyclohexylamino)-1-propanesulfonic acid), CAPSO (3-(cyclohexylamino)-2-hydroxy-1-propanesulfonic acid), EPPS (4-(2-hydroxyethyl)-1-piperazinepropanesulfonic acid), HEPES (4-(2-hydroxyethyl)piperazine-1-ethanesulfonic acid), MES (2-(N-morpholino)ethanesulfonic acid), MOPS (3-(N-morpholino)propanesulfonic acid), MOPSO (3-morpholino-2-hydroxypropanesulfonic acid), PIPES (1,4-piperazinediethanesulfonic acid), TAPS (N-[tris(hydroxymethyl)methyl]-3-aminopropanesulfonic acid), TAPSO (2-hydroxy-3-[tris(hydroxymethyl)methylamino]-1-propanesulfonic acid), TES (N-[tris(hydroxymethyl)methyl]-2-aminoethanesulfonic acid), bicine (N,N-Bis(2-hydroxyethyl)glycine), tricine (N-[Tris(hydroxymethyl)methyl]glycine), tris (tris(hydroxymethyl)aminomethane) and bis-tris (2-[Bis(2-hydroxyethyl)amino]-2-(hydroxymethyl)-1,3-propanediol). Certain embodiments contemplated herein, including a number of those set forth in Tables X, may feature a formulation having a pH of about 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9 or 9.0, where "about" may be understood to represent quantitative variation that may be more or less than the recited pH value by less than 1, preferably less than 0.5, preferably less than 0.25, and more preferably less than 0.1 pH unit.

### 2. Chelating Agents

Chelating agents or chelators may, according to certain embodiments, be included in the presently described composition for substantially stable storage of viable, intact cells in a blood sample, and are known to those familiar with the art for their ability to complex with and hinder the reactivity of metal cations. Exemplary chelating agents include diethylenetriaminepentaacetic acid (DTPA), ethylenediaminetetraacetic acid (EDTA), ethylene glycol tetraacetic acid (EGTA), trans-1,2-diaminocyclohexane-N,N,N',N'-tetraacetic acid (CDTA), 1,2-bis(2-aminophenoxy)ethane-N,N,N',N'-tetraacetic acid (BAPTA), 1,4,7,10-tetraazacyclododecane-1,4,7,10-tetraacetic acid (DOTA), N-(2-hydroxyethyl)ethylenediamine-N,N',N'-triacetic acid, sodium gluconate, and nitrilotriacetic acid (NTA). One chelating agent is sodium gluconate and is present at a concentration of about 1.0 - 50 mM, more preferably about 10 - 40 mM, and even more preferably about 25 mM, where "about" may be understood to represent quantitative variation that may be more or less than the recited amount by less than 25%, more preferably less than 20%, and more preferably less than 15%, 10%, 5% or 1%.

**TABLE 1**

| EXEMPLARY DEHYDRATION FORMULATIONS FOR SUBSTANTIALLY DRY STORAGE OF FUNCTIONAL CELLS AT AMBIENT TEMPERATURES | |
|---|---|
| MCS1 | 10 mM Tris-HCl (pH 7.5), 5 mM KCl, 65 mM NaCl, 150 mM Trehalose, 1% PVA, pH 7.5 |
| MCS2 | 10 mM HEPES, 5 mM KCl, 65 mM NaCl, 150 mM Trehalose, 1% PVA, pH 7.25 |
| MCS3 | 10mM HEPES, 5 mM KCl, 65 mM NaCl, 150 mM Trehalose, 1% PVA, 30 µM MLS-0315763.002 pH 7.4 |
| MCS4 | 10 mM HEPES, 5 mM KCl, 65 mM NaCl, 150 mM Trehalose, 1% PVA, 30 µM BIM-0306464.0001, pH 7.2 |
| MCS5 | 10 mM HEPES, 5 mM KCl, 65 mM NaCl, 150 mM Trehalose, 1% PVA, 30 µM BIM-0306464.0001 pH 7.2 |
| MCS6 | 10 mM HEPES, 5 mM KCl, 65 mM NaCl, 150 mM Trehalose, 1% PVA , 30 µM BIM-0306464.0001, pH 7.2 |
| MCS7 | 10 mM HEPES, 5 mM KCl, 65 mM NaCl, 150 mM Trehalose, 1% PVA, 30 µM salubrinal, pH 7.3 |
| MCS8 | 10 mM HEPES, 5 mM KCl, 65 mM NaCl, 100 mM Trehalose, 1% PVA, 30 µM Caspase-1 Inhibitor II pH 7.3 |
| MCS9 | 10 mM HEPES, 5 mM KCl, 65 mM NaCl, 100 mM Trehalose, 1% PVA, 30 µM Q-VD-Oph, pH 7.3 |
| MCS10 | 50 mM Tris-HCl (pH 8.0), 1% PVA, 10 % sucrose, 6% melezitoses |
| MCS11 | 50 mM Tris-HCl (pH 8), 1% PVA, 10 % sucrose, 6% melezitoses, 30 µM salubrinal |
| MCS12 | 50 mM Tris-HCl (pH 8), 1% PVA, 10 % sucrose, 6% melezitoses, 30 µM salubrinal, 5 µM arbutin |
| MCS13 | 10 mM HEPES, 5 mM KCl, 65 mM NaCl, 100 mM Trehalose, 1% PVA, 30 µM salubrinal, 5 µM arbutin, pH 7.3 |
| MCS14 | 10 mM HEPES, 5 mM KCl, 65 mM NaCl, 150 mM Trehalose, 1% PVA, 30 µM salubrinal, pH 7.3 |
| MCS15 | 10 mM Tris-HCl (pH 7.5), 2% HES, 100 mM Trehalose, 5 mM KCl, 60 mM NaCl, 30 µM salubrinal, pH 7.26 |
| MCS16 | 10 mM Ala-Glutamine, 5 mM EDTA, 10 mM Tris-HCl (pH 7.5), 1% PVA, pH 6.9 |
| MCS17 | 50 mM Ala-Glutamine, 5 mM EDTA, 10 mM Tris-HCl (pH 7.5), 1% PVA, pH 6.8 |
| MCS18 | 100 mM Ala-Glutamine, 5 mM EDTA, 10 mM Tris-HCl (pH 7.5), 1% PVA, pH 6.8 |
| MCS19 | 10 mM L-Glutamine, 5 mM EDTA, 10 mM Tris-HCl (pH 7.5), 1% PVA |
| MCS20 | 50 mM L-Glutamine, 5 mM EDTA, 10 mM Tris-HCl (pH 7.5), 1% PVA |
| MCS21 | 100 mM L-Glutamine, 5 mM EDTA, 10 mM Tris-HCl (pH 7.5), 1% PVA |
| MCS22 | 100 mM Ala-Glutamine, 5 mM EDTA, 10 mM Tris-HCl (pH 7.5), 1% PVA, 30 µM salubrinal, pH 6.8 |
| MCS23 | 100 mM Ala-Glutamine, 5 mM EDTA, 10 mM Tris-HCl (pH 7.5), 1% PVA, ) 30 µM MLS-0315763.002 (22.5 µM total DMSO), pH 6.8 |
| MCS24 | 100 mM Ala-Glutamine, 5 mM EDTA, 10 mM Tris-HCl (pH 7.5), 1% PVA, 30 µM BIM-0306464.0001, pH 6.8 |
| MCS25 | 100 mM Ala-Glutamine, 5 mM EDTA, 10 mM Tris-HCl (pH 7.5), 1% PVA, 30 µM BIM-0306464.0001, pH 6.75 |
| MCS26 | 100 mM Ala-Glutamine, 5 mM EDTA, 10 mM Tris-HCl (pH 7.5), 1% PVA, 30 µM BIM-0306464.0001, pH 6.8 |
| MCS27 | 100 mM Ala-Glutamine, 5 mM EDTA, 100 mM Trehalose, 10 mM |
| MCS28 | 200 mM L-Glutamine, 5 mM EDTA, 10 mM Tris-HCl (pH 7.5), 1% PVA, pH 6.8 |
| MCS29 | 200 mM L-Glutamine, 5 mM EDTA, 10 mM Tris-HCl (pH 7.5), 1% PVA, 30 µM MLS-0315763.002 (22.5 µM total DMSO), pH 6.86 |
| MCS30 | 200 mM L-Glutamine, 5 mM EDTA, 10 mM Tris-HCl (pH 7.5), 1% PVA, 30 µM BIM-0306464.0001, pH 6.9 |
| MCS31 | 200 mM L-Glutamine, 5 mM EDTA, 10 mM Tris-HCl (pH 7.5), 1% PVA, 30 µM BIM-0306464.0001, pH 7.05 |
| MCS32 | 200 mM L-Glutamine, 5 mM EDTA, 10 mM Tris-HCl (pH 7.5), 1% PVA, 30 µM BIM-0306464.0001, pH 7.0 |
| MCS33 | 200 mM L-Glutamine, 5 mM EDTA, 10 mM Tris-HCl (pH 7.5), 1% PVA, 30 µM salubrinal, pH 6.8 |
| MCS34 | 100 mM L-Glutamine, 5 mM EDTA, 100 mM Trehalose, 10 mM Tris-HCl (pH 7.5), 1% PVA, pH 6.9 |
| MCS35 | 100 mM L-Glutamine, 5 mM EDTA, 10 mM Tris-HCl (pH 7.5), 1% PVA, 30 µM salubrinal, pH 6.9 |
| MCS36 | 100 mM L-Glutamine, 5 mM EDTA, 100 mM Trehalose, 10 mM Tris-HCl (pH 7.5), 1% PVA, pH 7.08 |
| MCS37 | 100 mM Ala-Glutamine, 5 mM EDTA, 100 mM Trehalose, 10 mM Tris-HCl (pH 7.5), 1% PVA, 30 µM salubrinal, pH 6.86 |
| MCS38 | 100 mM L-Glutamine, 5 mM EDTA, 10 mM Tris-HCl (pH 7.5), 5 mM L-proline, 1% PVA, pH 6.95 |
| MCS39 | 100 mM L-Glutamine, 5 mM EDTA, 10 mM Tris-HCl (pH 7.5), 5 mM trans-4-hydroxy-L-proline, 1% PVA, pH 6.85 |
| MCS40 | 100 mM L-Glutamine, 5 mM EDTA, 10 mM Tris-HCl (pH 7.5), 5 mM Glycine, 1% PVA, pH 6.96 |
| MCS41 | 100 mM Ala-Glutamine, 5 mM EDTA, 10 mM Tris-HCl (pH 7.5), 5 mM Proline, 1% PVA, pH 6.8 |
| MCS42 | 100 mM Ala-Glutamine, 5 mM EDTA, 10 mM Tris-HCl (pH 7.5), 5 mM trans-4-hydroxy L-proline, 1% PVA, pH 6.8 |
| MCS43 | 100 mM Ala-Glutamine, 5 mM EDTA, 10 mM Tris-HCl (pH 7.5), 5 mM Glycine, 1% PVA, pH 6.8 |
| MCS44 | 100 mM L-Glutamine, 25 mM EDTA, 10 mM Tris-HCl (pH 7.5), 1% PVA, 30 µM salubrinal, pH 6.85 |
| MCS45 | 100 mM L-Glutamine, 5 mM EDTA, 10 mM Tris-HCl (pH 7.5), 1% PVA, 30 µM TDCA, pH 6.7 |
| MCS46 | 100 mM Ala-Glutamine, 5 mM EDTA, 10 mM Tris-HCl (pH 7.5), 1% PVA, 30 µM TDCA, ,pH 6.83 |
| MCS47 | 100 mM L-Glutamine, 5 mM EDTA, 10 mM Tris-HCl (pH 7.5), 1% PVA, 30 µM TDCA, pH 7.03 |
| MCS48 | 100 mM Ala-Glutamine, 5 mM EDTA, 10 mM Tris-HCl (pH 7.5), 1% PVA, 30 µM TDCA, pH 7.03 |
| MCS49 | 100 mM L-Glutamine, 5 mM EDTA, 10 mM Tris-HCl (pH 7.5), 1% PVA, 30 µM Caspase-1 Inhibitor II, pH 7.06 |
| MCS50 | 100 mM L-Glutamine, 5 mM EDTA, 10 mM Tris-HCl (pH 7.5), 1% PVA, 30 µM Q-VD-Oph, pH 7.0 |
| MCS51 | 100 mM Ala-Glutamine, 5 mM EDTA, 10 mM Tris-HCl (pH 7.5), 30 µM salubrinal, 1% PVA |
| MCS52 | 100 mM Ala-Glutamine, 5 mM EDTA, 10 mM Tris-HCl (pH 7.5), 1% PVA, 30 µM Caspase-1 Inhibitor II |
| MCS53 | 100 mM Ala-Glutamine, 5 mM EDTA, 10 mM Tris-HCl (pH 7.5), 30 µM Q-VD-Oph, 1% PVA |
| MCS54 | 100 mM Ala-Glutamine, 5 mM EDTA, 10 mM Tris-HCl (pH 7.5), 100 mM Trehalose, 30 µM Q-VD-Oph, 1% PVA |
| MCS55 | 100 mM Glutathione, 5 mM EDTA, 10 mM Tris-HCl (pH 7.5), 1% PVA, 5 mM Betaine, pH 6.8 |
| MCS56 | 100 mM Ala-Glutamine, 5 mM EDTA, 10 mM Tris-HCl (pH 7.5), 1% PVA |
| MCS57 | 10 mM HEPES, 5 mM KCl, 65 mM NaCl, 100 mM hydroxyectoine, 1% PVA, pH 7.0 |
| MCS58 | 10 mM HEPES, 5 mM KCl, 65 mM NaCl, 100 mM hydroxyectoine, 1% PVA, 30 µM salubrinal, pH 6.93 |
| MCS59 | 50 mM hydroxyectoine, 5 mM EDTA, 10 mM Tris-HCl (pH 7.5), 1% PVA, pH 7.15 |
| MCS60 | 100 mM hydroxyectoine, 5 mM EDTA, 10 mM Tris-HCl (pH 7.5), 1% PVA, pH 7.2 |
| MCS61 | 200 mM hydroxyectoine, 5 mM EDTA, 10 mM Tris-HCl (pH 7.5), 1% PVA, pH 7.12 |
| MCS62 | 100 mM hydroxyectoine, 5 mM EDTA, 10 mM Tris-HCl (pH 7.5), 1% HES, pH 7.14 |
| MCS63 | 100 mM hydroxyectoine, 5 mM EDTA, 10 mM Tris-HCl (pH 7.5), 1% HES, 1% PVA, pH 7.06 |
| MCS64 | 50 mM hydroxyectoine, 5 mM EDTA, 10 mM Tris-HCl (pH 7.5), 5 mM hydroxy proline, 1% PVA, pH 7.09 |
| MCS65 | 50 mM hydroxyectoine, 5 mM EDTA, 10 mM Tris-HCl (pH 7.5), 5 mM Betaine, 1% PVA, pH 6.94 |
| MCS66 | 100 mM hydroxyectoine, 5 mM EDTA, 10 mM Tris-HCl (pH 7.5), 1% PVA, 30 µM salubrinal, pH 7.12 |
| MCS67 | 50 mM hydroxyectoine, 5 mM EDTA, 10 mM Tris-HCl (pH 7.5), 50 mM Glutathione, 1% PVA, 30 µM salubrinal, pH 6.93 |
| MCS68 | 50 mM hydroxyectoine, 5 mM EDTA, 10 mM Tris-HCl (pH 7.5), 50 mM L-Glutamine, 1% PVA, pH 7.2 |
| MCS69 | 50 mM hydroxyectoine, 5 mM EDTA, 10 mM Tris-HCl (pH 7.5), 50 mM L-Glutamine, 1% PVA, 30 µM salubrinal, pH 7.0 |
| MCS70 | 10 mM HEPES, 5 mM KCl, 50 mM NaCl, 100 mM Trehalose, 50 mM hydroxyectoine, 1% PVA, 30 µM salubrinal, pH 7.12 |
| MCS71 | 10 mM HEPES, 5 mM KCl, 50 mM NaCl, 100 mM Trehalose, 50 mM hydroxyectoine, 1% PVA, pH 7.07 |
| MCS72 | 50 mM hydroxyectoine, 5 mM EDTA, 10 mM Tris-HCl (pH 7.5), 50 mM ALA-GLN, 1% PVA, pH 6.96 |
| MCS73 | 100 mM Glutathione, 5 mM EDTA, 10 mM Tris-HCl (pH 7.5), 1% PVA, pH 6.8 |
| MCS74 | 100 mM Glutathione, 5 mM EDTA, 10 mM Tris-HCl (pH 7.5), 1% HES, pH 6.8 |
| MCS75 | 100 mM Glutathione, 5 mM EDTA, 50 mM Trehalose, 10 mM Tris-HCl (pH 7.5), 1% PVA, pH 7.18 |
| MCS76 | 100 mM Glutathione, 5 mM EDTA, 10 mM Tris-HCl (pH 7.5), 1% PVA, 30 µM salubrinal, pH 6.8 |
| MCS77 | 100 mM Glutathione, 5 mM EDTA, 10 mM Tris-HCl (pH 7.5), 1% PVA, 30 µM TDCA, pH 6.8 |
| MCS78 | 100 mM Glutathione, 5 mM EDTA, 10 mM Tris-HCl (pH 7.5), 1% PVA, 30 µM MLS-0315763.002, pH 6.8 |
| MCS79 | 100 mM Glutathione, 5 mM EDTA, 10 mM Tris-HCl (pH 7.5), 1% PVA, 30 µM BIM-0306464.0001, pH 6.8 |
| MCS80 | 100 mM Glutathione, 5 mM EDTA, 10 mM Tris-HCl (pH 7.5), 1% PVA, 30 µM BIM-0306464.0001, pH 6.8 |
| MCS81 | 100 mM Glutathione, 5 mM EDTA, 10 mM Tris-HCl (pH 7.5), 1% PVA, 30 µM BIM-0306464.0001, pH 6.8 |
| MCS82 | 10 mM HEPES, 5 mM KCl, 50 mM NaCl, 100 mM Trehalose, 50 mM Glutamine, 1% PVA, pH 7.3 |
| MCS83 | 10 mM HEPES, 5 mM KCl, 50 mM NaCl, 100 mM Trehalose, 50 mM Glutamine, 1% PVA, 30 µM salubrinal, pH 7.3 |
| MCS84 | 10 mM HEPES, 5 mM KCl, 50 mM NaCl, 100 mM Hydroxyectoine, 50 mM Glutamine, 1% PVA, pH 7.3 |
| MCS85 | 10 mM HEPES, 5 mM KCl, 50 mM NaCl, 100 mM Hydroxyectoine, 50 mM Glutamine, 1% PVA, pH 7.3 |
| MCS86 | 100 mM Glutamic acid, 5 mM EDTA, 10 mM NH₄Cl, 10 mM Tris-HCl (pH 7.5), 1% PVA |
| MCS87 | 5 mM Proline, 10 mM Tris-HCl (pH 7.5), 5 mM EDTA, 1% PVA |
| MCS88 | 100 mM Ala-Glutamine, 10 mM Tris-HCl (pH 7.5), 5 mM EDTA, 1 % PVA |
| MCS89 | 100 mM Ala-Glutamine, 5 mM Proline, 10 mM Tris-HCl (pH 7.5), 1% PVA |
| MCS90 | 100 mM Ala-Glutamine, 5 mM Proline, 10 mM Tris-HCl (pH 7.5), 5 mM EDTA |
| MCS91 | 100 mM Betaine5 mM Proline, 10 mM Tris-HCl (pH 7.5), 5 mM EDTA, 1% PVA |
| MCS92 | 100 mM Ala-Glutamine, 50 mM Proline, 10 mM Tris-HCl (pH 7.5), 5 mM EDTA, 1% PVA |
| MCS93 | 100 mM Ala-Glutamine, 5 mM Proline, 10 mM HEPES (pH 7.5)5 mM EDTA, 1% PVA |
| MCS94 | 100 mM Ala-Glutamine, 5 mM Proline, 10 mM Tris-HCl (pH 7.5), 5 mM EDTA, 1% HES |
| MCS95 | 100 mM Ala-Glutamine, 5 mM Proline, 10 mM Tris-HCl (pH 7.5), 5 mM EDTA, 1% PEO |
| MCS96 | 100 mM Ala-Glutamine, 5 mM Proline, 10 mM Tris-HCl (pH 7.5), 5 mM EDTA1% PEG-8000 |
| MCS97 | 100 mM Ala-Glutamine, 5 mM Proline, 10 mM Tris-HCl (pH 7.5), 5 mM EDTA1% PVP-10 |
| MCS100 | 10 mM Tris-HCl (pH 7.5), pH 6.8 |
| MCS101 | 10 mM Tris-HCl (pH 7.5), 1% PEG8000, 1% HES, 1% PVA, 1% PEO, 1% PVP-10, pH 6.8 |
| MCS102 | 10 mM Tris-HCl (pH 7.5), 1% PEG8000, 100 mM Betaine, 5mM EDTA, 1% PVP-10, pH 8 |
| MCS103 | 10 mM Tris-HCl (pH 7.5), 5 mM EDTA, 1% HES, 10 mM Proline, 1% PEO, pH 8 |
| MCS 104 | 10 mM Tris-HCl (pH 7.5), 5 mM EDTA, 1% PVA, 100 mM Betaine, 10 mM Proline, 1% PEO, 1% PVP-10, pH 6.8 |
| MCS105 | 10 mM Tris-HCl (pH 7.5), 100 mM Betaine, 10 mM Proline, 1% PVA, 1% HES, 1% PEG8000, pH 8 |
| MCS106 | 100 mM Ala-Glutamine, 10 mM Tris-HCl (pH 7.5), 100 mM Betaine, 10 mM Proline, 1% PEO, 1% PVP-10, pH 6.8 |
| MCS107 | 100 mM Ala-Glutamine, 10 mM Tris-HCl (pH 7.5), 100 mM Betaine, 5 mM EDTA, 1% PVA, 1% HES, pH 6.8 |
| MCS108 | 100 mM Ala-Glutamine, 10 mM Tris-HCl (pH 7.5), 5 mM EDTA, 1% PVA, 1% PEO, 1% PEG8000, pH 8 |
| MCS109 | 100 mM Ala-Glutamine, 10 mM Tris-HCl (pH 7.5), 100 mM Betaine, 10 mM Proline, 1% PEO, 1% PEG8000, pH 6.8 |
| MCS110 | 100 mM Ala-Glutamine, 10 mM Tris-HCl (pH 7.5), 10 mM Proline, 1% PVA, 1% PVP-10, pH 8 |
| MCS111 | 10 mM Ala-Glutamine, 10 mM Tris-HCl (pH 7.5), 5 mM EDTA, 1% HES, 10 mM Proline, 1%PEG8000, 1%PVP-10, pH 6.8 |
| MCS112 | 10 mM ALA-GLN, 10 mM Betaine, 10 mM Tris-HCl (pH 7.5), 1 mM EDTA,, pH6 |
| MCS113 | 10 mM ALA-GLN, 80 mM Betaine, 10 mM Tris-HCl (pH 7.5), 4.25 mMEDTA,0.75% PVA, 0.75% HES,pH7 |
| MCS114 | 10 mM ALA-GLN, 150 mM Betaine, 10 mM Tris-HCl (pH 7.5), 7.5 mMEDTA,1.5% PVA, 1.5% HES,pH8 |
| MCS115 | 80 mM ALA-GLN, 10 mM Betaine, 10 mM Tris-HCl (pH 7.5), 4.25 mMEDTA,0.75% PVA, 1.5% HES,pH6 |
| MCS116 | 80 mM ALA-GLN, 80 mM Betaine, 10 mM Tris-HCl (pH 7.5), 7.5 mMEDTA,1.5% PVA, pH7 |
| MCS117 | 80 mM ALA-GLN, 150 mM Betaine, 10 mM Tris-HCl (pH 7.5), 1 mMEDTA,,0.75% HES,pH8 |
| MCS118 | 150 mM ALA-GLN, 10 mM Betaine, 10 mM Tris-HCl (pH 7.5), 1 mMEDTA,1.5% PVA, 0.75% HES,pH7 |
| MCS119 | 150 mM ALA-GLN, 80 mM Betaine, 10 mM Tris-HCl (pH 7.5), 4.25 mMEDTA,,1.5% HES,pH8 |
| MCS120 | 150 mM ALA-GLN, 150 mM Betaine, 10 mM Tris-HCl (pH 7.5), 7.5 mM EDTA,0.75% PVA,pH6 |
| MCS121 | 10 mM ALA-GLN, 10 mM Betaine, 10 mM Tris-HCl (pH 7.5), 7.5 mMEDTA,0.75% PVA, 0.75% HES,pH8 |
| MCS122 | 10 mM ALA-GLN, 80 mM Betaine, 10 mM Tris-HCl (pH 7.5), 1 mM EDTA,1.5% PVA, 1.5% HES,pH6 |
| MCS123 | 10 mM ALA-GLN, 150 mM Betaine, 10 mM Tris-HCl (pH 7.5), 4.25 mMEDTA, pH7 |
| MCS124 | 80 mM ALA-GLN, 10 mM Betaine, 10 mM Tris-HCl (pH 7.5), 7.5 mMEDTA,,1.5% HES,pH7 |
| MCS125 | 80 mM ALA-GLN, 80 mM Betaine, 10 mM Tris-HCl (pH 7.5), 1 mMEDTA,0.75% PVA,pH8 |
| MCS126 | 80 mM ALA-GLN, 150 mM Betaine, 10 mM Tris-HCl (pH 7.5), 4.25 mMEDTA,1.5% PVA, 0.75% HES,pH6 |
| MCS127 | 150 mM ALA-GLN, 10 mM Betaine, 10 mM Tris-HCl (pH 7.5), 4.25 mMEDTA,1.5% PVA,pH8 |
| MCS128 | 150 mM ALA-GLN, 80 mM Betaine, 10 mM Tris-HCl (pH 7.5), 7.5 mMEDTA,,0.75% HES,pH6 |
| MCS129 | 150 mM ALA-GLN, 150 mM Betaine, 10 mM Tris-HCl (pH 7.5), 1 mMEDTA,0.75% PVA, 1.5% HES,pH7 |
| MCS130 | 10 mM Tris-HCl (pH 7.5), pH 6 |
| MCS131 | 10 mM Tris-HCl (pH 7.5), pH 8 |
| MCS132 | 150 mM ALA-GLN, 10 mM Tris-HCl (pH 7.5), 150 mM Betaine, pH 6 |
| MCS133 | 150 mM ALA-GLN, 10 mM Tris-HCl (pH 7.5), 150 mM Betaine pH 8 |
| MCS134 | 150 mM ALA-GLN, 10 mM TrisHCl, pH 6.0 |
| MCS136 | 150 mM Betaine, 10 mM TrisHCl, pH 6.0 |
| MCS137 | 150 mM Betaine, 10 mM TrisHCl, pH 8.0 |
| MCS138 | 10 mM Tris-HCl (pH 7.5), 1.5% PVA, pH 6.0 |
| MCS139 | 10 mM Tris-HCl (pH 7.5), 1.5% PVA, pH 8.0 |
| MCS140 | 150 mM ALA-GLN, 10 mM Tris-HCl (pH 7.5), 150 mM Betaine, 1.5% PVA, pH 6 |
| MCS141 | 150 mM ALA-GLN, 10 mM Tris-HCl (pH 7.5), 150 mM Betaine, 1.5% PVA, pH 8 |
| MCS142 | 150 mM ALA-GLN, 10 mM Tris-HCl (pH 7.5), 1.5% PVA, pH 6 |
| MCS143 | 150 mM ALA-GLN, 10 mM Tris-HCl (pH 7.5), 1.5% PVA, pH 8 |
| MCS144 | 10 mM Tris-HCl (pH 7.5), 150 mM Betaine, 1.5% PVA, pH 6 |
| MCS145 | 10 mM Tris-HCl (pH 7.5), 150 mM Betaine, 1.5% PVA, pH 8 |
| MCS146 | 10 mM Tris-HCl (pH 7.5), 1.5% HES, pH 6.0 |
| MCS147 | 10 mM Tris-HCl (pH 7.5), 1.5% HES, pH 8.0 |
| MCS148 | 150 mM ALA-GLN, 10 mM Tris-HCl (pH 7.5), 150 mM Betaine, 1.5% HES, pH 6 |
| MCS149 | 150 mM ALA-GLN, 10 mM Tris-HCl (pH 7.5), 150 mM Betaine, 1.5% HES, pH 8 |
| MCS150 | 150 mM ALA-GLN, 10 mM Tris-HCl (pH 7.5), 1.5% PVA, pH 6 |
| MCS151 | 150 mM ALA-GLN, 10 mM Tris-HCl (pH 7.5), 1.5% PVA, pH 8 |
| MCS152 | 10 mM Tris-HCl (pH 7.5), 150 mM Betaine, 1.5% PVA, pH 6 |
| MCS153 | 10 mM Tris-HCl (pH 7.5), 150 mM Betaine, 1.5% PVA, pH 8 |
| MCS154 | 300 mM ALA-GLN, 10 mM Tris-HCl (pH 7.5), 3% HES pH 8 |
| MCS155 | 82.5 mM ALA-GLN, 10 mM Tris-HCl (pH 7.5),15 mM Betaine, 0.15% PVA, pH 6 |
| MCS156 | 82.5 mM ALA-GLN, 10 mM Tris-HCl (pH 7.5), 150 mM Betaine, 0.15% PVA, pH 6 |
| MCS157 | 82.5 mM ALA-GLN, 10 mM Tris-HCl (pH 7.5),15 mM Betaine, 1.5% PVA, pH 6 |
| MCS158 | 82.5 mM ALA-GLN, 10 mM Tris-HCl (pH 7.5), 150 mM Betaine, 1.5% PVA, pH 6 |
| MCS159 | 15 mM ALA-GLN, 10 mM Tris-HCl (pH 7.5), 15 mM Betaine, 0.825% PVA, pH 6 |
| MCS160 | 150 mM ALA-GLN, 10 mM Tris-HCl (pH 7.5), 15 mM Betaine, 0.825% PVA, pH 6 |
| MCS161 | 15 mM ALA-GLN, 10 mM Tris-HCl (pH 7.5), 150 mM Betaine, 0.825% PVA, pH 6 |
| MCS162 | 150 mM ALA-GLN, 10 mM Tris-HCl (pH 7.5), 150 mM Betaine, 0.825% PVA, pH 6 |
| MCS163 | 15 mM ALA-GLN, 10 mM Tris-HCl (pH 7.5),82.5 mM Betaine, 0.15% PVA, pH 6 |
| MCS164 | 15 mM ALA-GLN, 10 mM Tris-HCl (pH 7.5),82.5 mM Betaine, 1.5% PVA, pH 6 |
| MCS165 | 150 mM ALA-GLN, 10 mM Tris-HCl (pH 7.5),82.5 mM Betaine, 0.15% PVA, pH 6 |
| MCS166 | 150 mM ALA-GLN, 10 mM Tris-HCl (pH 7.5),82.5 mM Betaine, 1.5% PVA, pH 6 |
| MCS167 | 82.5 mM ALA-GLN, 10 mM Tris-HCl (pH 7.5),82.5 mM Betaine, 0.825% PVA, pH 6 |
| MCS168 | 150 mM ALA-GLN, 10 mM Tris-HCl (pH 7.5), 150 mM Betaine, 0.15% PVA, pH 6 |
| MCS169 | 15 mM ALA-GLN, 10 mM Tris-HCl (pH 7.5),15 mM Betaine, 1.5% PVA, pH 6 |
| MCS170 | 150 mM ALA-GLN, 10 mM Tris-HCl (pH 7.5), 150 mM Betaine, pH 6 |
| MCS171 | 150 mM ALA-GLN, 10 mM Bis-Tris-HCl, 150 mM Betaine, pH 6 |
| MCS172 | 150 mM ALA-GLN, 10 mM MES, 150 mM Betaine, pH 6 |
| MCS173 | 300 mM ALA-GLN, 10 mM Tris-HCl (pH 7.5), pH 6 |
| MCS174 | 300 mM ALA-GLN, 10 mM Bis-Tris-HCl, pH 6 |
| MCS175 | 300 mM ALA-GLN, 10 mM MES, pH 6 |
| MCS176 | 300 mM Betaine, 10 mM Tris-HCl (pH 7.5), pH 6 |
| MCS177 | 300 mM Betaine, 10 mM Bis-Tris-HCl, pH 6 |
| MCS178 | 300 mM Betaine, 10 mM MES, pH 6 |
| MCS179 | 300 mM ALA-GLN, 10 mM Tris-HCl (pH 7.5), 300 mM Betaine, pH 6 |
| MCS180 | 300 mM ALA-GLN, 10 mM Bis-Tris-HCl, 300 mM Betaine, pH 6 |
| MCS181 | 300 mM ALA-GLN, 10 mM MES, 300 mM Betaine, pH 6 |
| MCS182 | 450 mM ALA-GLN, 10 mM Tris-HCl (pH 7.5), 450 mM Betaine, pH 6 |
| MCS183 | 450 mM ALA-GLN, 10 mM Bis-Tris-HCl, 450 mM Betaine, pH 6 |
| MCS184 | 450 mM ALA-GLN, 10 mM MES, 450 mM Betaine, pH 6 |
| MCS185 | 10 mM TrisHCl, 1% PVA, pH 6.0 |
| MCS186 | 75 mM ALA-GLN, 75 mM Betaine, 10 mM TrisHCl, pH 6.0 |
| MCS187 | 75 mM ALA-GLN, 10 mM TrisHCl, 0.5% PVA pH 6.0 |
| MCS188 | 75 mM Betaine, 10 mM TrisHCl, 0.5% PVA |
| MCS189 | 50 mM ALA-GLN, 50 mM Betaine, 10 mM TrisHCl, 0.33% PVA, pH 6.0 |
| MCS190 | 150 mM ALA-GLN, 10 mM Bis-Tris, pH 6.0 |
| MCS191 | 150 mM Betaine, pH 6.0 |
| MCS192 | 10 mM Bis-Tris, 1% PVA, pH 6.0 |
| MCS193 | 75 mM ALA-GLN, 75 mM Betaine, 10 mM Bis-Tris, pH 6.0 |
| MCS194 | 75 mM ALA-GLN, 10 mM Bis-Tris, 0.5 % PVA, pH 6.0 |
| MCS195 | 75 mM Betaine, 10 mM Bis-Tris, 0.5% PVA, pH 6.0 |
| MCS196 | 50 mM ALA-GLN, 50 mM Betaine, 10 mM Bis-Tris, 0.33% PVA, pH 6.0 |
| MCS197 | 150 mM ALA-GLN, 10 mM MES, pH 6.0 |
| MCS198 | 150 mM Betaine, 10 mM MES, pH 6.0 |
| MCS199 | 10 mM MES, 1% PVA, pH 6.0 |
| MCS200 | 75 mM ALA-GLN, 75 mM Betaine, 10 mM MES, pH 6.0 |
| MCS201 | 75 mM ALA-GLN, 10 mM MES, 0.5% PVA, pH 6.0 |
| MCS202 | 75 mM Betaine, 10 mM Mes, 0.5% PVA, pH 6.0 |
| MCS203 | 50 mM ALA-GLN, 50 mM Betaine, 10 mM MES, 0.33% PVA, pH 6.0 |

### DEHYDRATION PROCESS

The MCS dehydration formulations of the present invention are capable of maintaining the integrity of cell membranes and organelles as well as the general morphology of the cells during the dehydration process, in the presence or absence of a prior treatment with a predehydration formulation, such that upon rehydration of the cells after substantially dry stored the cells retain at least one functional property, e.g., cell viability, as the cells prior to dehydration.

Drying of the dehydrated cells can be determined, for example, by simple visual inspection to ensure all moisture has been evaporated or removed. In some embodiments, a moisture indicator may be preferably included to ascertain a degree of drying has been achieved. The time to substantially dry cells can vary depending on the reagents present in the MCS dehydration formulations. The cells are optimally dehydrated in a period of about one to three hours depending on formulation components and geometry of the vessel to which the cells are immobilized. The cells are dehydrated at a temperature range of about 32°C - 39°C, preferably about 37°C, and may be dehydrated in an incubator or under more controlled conditions using an environmental chamber to control temperature, oxygen levels and the relative humidity. By adjusting the relative humidity, the rate at which dehydration may be modulated. For instance, cells dehydrated in MCS formulations comprising a water-soluble polymer, e.g., PVA, will take a longer period to dehydrate so the relative humidity may be decreased to facilitate optimal dehydration times. In addition, the substantial dry storage of various stem cells is performed at 5% oxygen concentration to ensure the cells are maintained in a substantially immunologically undifferentiated state.

Other methods of dehydration may be employed that maintain active air movement above the cells while controlling temperature and humidity.

The substantially dry cells may be stored using a hermetically sealable cover or pouch so that the contents may be sealed for storage under similar climate conditions used to dehydrate the cells. The substantially dry stored cells are optimally stored at constant temperature, e.g., room temperature.

### APOPTOSIS INHIBITORS

In certain embodiments, the predehydration formulation, dehydration formulation and/or the rehydration formulation described herein comprises at least one apoptosis inhibitor. In certain embodiments, the at least one apoptosis inhibitor blocks the induction of cellular apoptosis. In other embodiments, the apoptosis inhibitor blocks at least one step in the ER stress pathway, and preferably is a reversible inhibitor.

In certain embodiments, the dehydration formulation comprises the at least one apoptosis inhibitor. In other embodiments, the predehydration formulation or the rehydration formulation comprise the at least one apoptosis inhibitor, and in yet other embodiments, the predehydration formulation and the rehydration formulation each comprise at least one apoptosis inhibitor. The at least one apoptosis inhibitor present in the predehydration formulation and the rehydration formulation may be the same or may be different. In certain other embodiments, the predehydration formulation, dehydration formulation and/or the rehydration formulation comprises at least two apoptosis inhibitors.

Apoptosis inhibitors, including those exemplified herein, are generally commercially available through a number of commercial manufacturers and suppliers including, but not limited to, Calbiochem, SelleckChem, Sigma-Aldrich, EMD Millipore, LCLabs, and medchemexpress, or may be synthesized using known methods, including those disclosed herein. The optimal concentration of each apoptosis inhibitor may be determined by titrating the amount of apoptosis inhibitor in the predehydration, dehydration and/or rehydration formulations, which is well within the purview of those skilled in the art.

Exemplary apoptosis inhibitors include:

### 1. PERK-eIF2-α Inhibitors

In certain embodiments, the at least one apoptosis inhibitor blocks the PERK-eIF2-α alpha pathway. Exemplary PERK-eIF2-α alpha pathway inhibitors are salubrinal. Sal-003 (3-phenyl-N-[2,2,2-trichloro-1-[(4-chlorophenyl)carbamothioylamino]ethyl]prop-2-enamide), GSK 2606414 (7-Methyl-5-(1-{[3-(trifluoromethyl)phenyl]acetyl}-2,3-dihydro-1H-indol-5-yl)-7H-pyrrolo[2,3d]pyrimidin-4-amine), GSK 2656157 (1-(5-(4-amino-7-methyl-7H-pyrrolo[2,3-d]pyrimidin-5-yl)-4-fluoroindolin-1-yl)-2-(6-methylpyridin-2-yl)ethanone) and ISRIB (trans-N,N'-(cyclohexane-1,4-diyl)bis(2-(4-chlorophenoxy)acetamide).

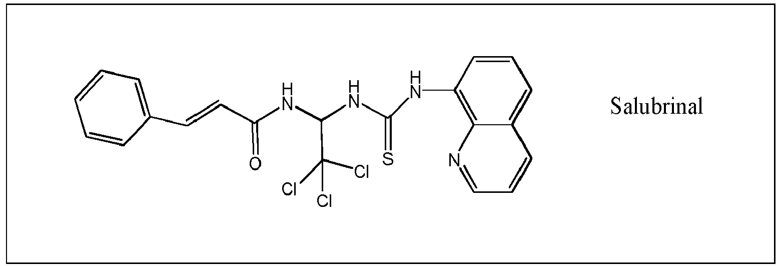

Salubrinal is a specific inhibitor of eIF2-α phosphatase enzymes. Salubrinal indirectly inhibits eIF2 as a result of reduced dephosphorylation of its α-subunit resulting in activation of stress response pathways usually triggered by events such as oxidative stress or buildup of unfolded protein in the endoplasmic reticulum.

In certain embodiments for substantially dry storage of cells for a period of greater than 24 hours, e.g., 48 or 120 hrs, salubrinal may be added to the predehydration and/or rehydration formulation at a concentration range of about between about 1 nM to about 2.0 µM, preferably between about 1 nM and 900 nM, more preferably about 10 nM and 250 nM, and even more preferably about 30 nM.

### 2. ASK1 Inhibitors

In other embodiments, the at least one apoptosis inhibitor is an ASK1 inhibitor, which blocks downstream activation of JNK and p38 MAP kinase. A variety of suitable ASK1 inhibitors are known (e.g., see US Patent Nos. 8,178,555; 8,378,108; 8,440,665 and 8,598,360) or are commercially available e.g., MLS-0315763 (National Institute of Health). Exemplary ASK1 inhibitors include, but are not limited to, benzodiazepinone inhibitors (Kim et al., (2009) J. Biol. Chem. 284:1593-1603), NDQI-1 and MLS-0315763.

In certain embodiments for substantially dry storage of cells for a period of greater than 24 hours, e.g., 48 or 120 hrs, NDQI-1 may be added to the predehydration and/or rehydration formulation at a concentration range of about between about 50 nM to about 3.0 µM, preferably between about 250 nM and 2.0 µM, more preferably about 400 nM and 2.0 µM, and even more preferred about 1.0 µM.

In certain other embodiments for substantially dry storage of cells for a period of greater than 24 hours, e.g., 48 or 120 hrs, MLS-0315763 may be added to the predehydration and/or rehydration formulation at a concentration range of about between about 1 nM to about 500 nM, preferably between about 1 nM and 250 nM, more preferably about 1 nM and 100 nM, and even more preferred about 10 nM.

### 3. NRF2-KEAP1 Inhibitors

In certain embodiments, the at least one apoptosis inhibitor blocks the NRF2-KEAP1 pathway.

Exemplary NRF2-KEAP1 pathway inhibitors include, but are not limited to, carnosic acid, tri- terpenoids, sulphoraphane, and tert-butylhydroquinone.

In certain embodiments for substantially dry storage of cells for a period of greater than 24 hours, e.g., 48 or 120 hrs, sulphoraphane may be added to the predehydration and/or rehydration formulation at a concentration range of about between about 50 nM to about 1.0 µM, preferably between about 50 nM and 500 nM, more preferably about 100 nM and 400 nM, and even more preferred about 220 nM.

### 4. JNK Inhibitors

In certain embodiments, the at least one apoptosis inhibitor is a JNK inhibitor. Any JNK inhibitor is contemplated for use in the formulations, compositions, methods of the present invention. JNK inhibitors are generally known to those skilled in the art (e.g., see US Patent Nos. 6,949,544; 7,129,242; 7,326,418, 8,143,271 and 8,530,480).

Exemplary JNK inhibitors include, but are not limited to, SP600125 (anthra[1-9-cd]pyrazol-6(2H)-one), JNK-IN-8 (3-[[4-(dimethylamino)-1-oxo-2-buten-1-yl]amino]-N-[3-methyl-4-[[4-(3-pyridinyl)-2-pyrimidinyl]amino]phenyl]-benzamide); and JNK-Inhibitor IX (N-(3-cyano-4,5,6,7-tetrahydrobenzo[b]thien-2-yl)- 1-naphthalenecarboxamide).

In still further embodiments, the JNK inhibitors are used in combination with a p38 MAP kinase inhibitor to block activation downstream of ASK1.

### 5. p38 MAP Kinase Inhibitors

In certain other embodiments, the at least one apoptosis inhibitor is a p38 MAP kinase inhibitor. p38 MAP kinase inhibitors are generally well known (e.g., see US Patent Nos. 7,521,460; 7,592,455; 7,728,013; and 7,795,256).

Exemplary p38 MAP kinase inhibitors include, but are not limited to, SB203580 (4-(4-(4-fluorophenyl)-2-(4-(methylsulfinyl)phenyl)-1H-imidazol-5-yl)pyridine), LY2228820 (5-(2-tert-butyl-4-(4-fluorophenyl)-1H-imidazol-5-yl)-3-neopentyl-3H-imidazo[4,5-b]pyridin-2-amine dimethanesulfonate), PD169316 (4-(4-fluorophenyl)-2-(4-nitrophenyl)-5-(4-pyridyl)-1H-imidazole), PH-797804 (3-(4-(2,4-difluorobenzyloxy)-3-bromo-6-methyl-2-oxopyridin-1(2H)-yl)-N,4-dimethylbenzamide), SB202190 (4-(4-(4-fluorophenyl)-5-(pyridin-4-yl)-1H-imidazol-2-yl)phenol), BIRB 796 (Doramapimod; 1-(3-tert-butyl-1-p-tolyl-1H-pyrazol-5-yl)-3-(4-(2-morpholinoethoxy)naphthalen-1-yl)urea), VX-702 (1-(5-carbamoyl-6-(2,4-difluorophenyl)pyridin-2-yl)-1-(2,6-difluorophenyl)urea), TAK-715 (N-[4-[2-ethyl-4-(3-methylphenyl)-5-thiazolyl]-2-pyridinyl]-benzamide.

In certain other embodiments, the predehydration formulation and/or the rehydration comprises a JNK inhibitor and p38 MAP kinase inhibitor to block downstream ASK1-dependent signaling.

### 6. GSK3 Inhibitors

In certain embodiments, the predehydration and/or rehydration formulations comprise an apoptosis inhibitor that blocks GSK3. A variety of GSK3 inhibitor are suitable for use in the formulations and methods described herein. GSK3 inhibitors are well known to those skilled in the art (e.g., see US Patent Nos. 6,057,117; 6,153,618; 6,417,185; 6,465,231; 6,489,344; 6,608,632; 6,800,632; 6,949,547; 7,045,519; 7,037, 918; 7,425,557; 8,143,271 and 8,664,244) and a number of GSK3 inhibitors are commercially available, e.g., CHIR98014, N-6-[2-[[4-(2,4-dichlorophenyl)-5-(1H-imidazol-2-yl)-2-pyrimidinyl]amino]ethyl]-3-nitro-2,6-pyridinediamine (Selleckchem.com; Catalog No. S2745) and valproic acid (Sigma-Aldrich, St. Louis, MO; Catalog No. P4543).

Particularly preferred GSK3 inhibitors include, but are not limited to, CHIR98014, Valproate, CT 99021 and CT 20026.

In certain embodiments for substantially dry storage of cells for a period of greater than 24 hours, e.g., 48 or 120 hrs, CHIR98014 may be added to the predehydration and/or rehydration formulation at a concentration range of about between about 0.25 µM to about 3.0 µM, preferably between about 0.5 µM and 2.75 µM, more preferably about 1.0 µM and 2.0 µM, and even more preferred about 1.25 µM.

### 7. IRE-1 Inhibitors

In certain embodiments, the predehydration, dehydration and/or rehydration formulations comprises an apoptosis inhibitor that blocks IRE1. IRE1 inhibitors are known (e.g., see US Patent Nos. 8,372,861).

Exemplary IRE1 inhibitors include, but are not limited to, IRE1 Inhibitor I (N-[(2-hydroxynaphthalen-1-yl)methylidene]thiophene-2-sulfonamide), IRE1 Inhibitor II (3'-formyl-4'-hydroxy-5'-methoxybiphenyl-3-carboxamide), and IRE1 Inhibitor III (8-formyl-7-hydroxy-4-methylcoumarin, 7-hydroxy-4-methyl-2-oxo-2H-chromene-8-carbaldehyde).

### 8. Caspase-1 Inhibitors

In certain embodiments, the at least one apoptosis inhibitor that is a caspase-1 inhibitor.

Exemplary caspase-1 inhibitors for use in the formulations, compositions and methods described herein include, but are not limited to, Caspase-1 Inhibitor II (Ac-YVAD-chloromethyl ketone), N-(2-Quinolyl)valyl-aspartyl-(2,6-difluorophenoxy)methyl ketone (in which the aspartyl residue is a- methylated or non-a-methylated), VX-765 ((S)-1-((S)-2-(4-amino-3-chlorobenzamido)-3,3-dimethylbutanoyl)-N-((2R,3S)-2-ethoxy-5-oxo-tetrahydrofuran-3-yl)pyrrolidine-2-carboxamide) and ZVAD-fluoromethyl ketone.

### 9. Calpain Inhibitors

In certain embodiments, the at least one apoptosis inhibitor is a calpain inhibitor. There are at least 15 different isoforms of calpain (Calpain 1-15). Calpain inhibitors are well known to those skilled in the art (e.g., see U.S. Patent Nos. 5,541,290; 6,448,245; 7,001,770; 7,476,754 and 7,932,266). The predehydration formulation and/or the rehydration formulation of the present invention may contain any suitable calpain inhibitor or combination of calpain inhibitors.

Particularly preferred calpain inhibitors for use in the formulations, compositions and methods described herein include, but are not limited to, Calpain Inhibitor I (N-Acetyl-Leu-Leu-Norleucine-CHO), Calpain Inhibitor II (N-Acetyl-Leu-Leu-Met), Calpain Inhibitor III (Z-Val-Phe-CHO), Calpain Inhibitor IV (Z-Leu-Leu-Tyr-CH₂F), Calpain Inhibitor V (Morpholinoureidyl;-Val-homophenylalanine-CH₂F), Calpain Inhibitor VI (4-Fluorophenylsulfonyl-Val-Leu-CHO), Calpain Inhibitor X (Z-Leu-α-aminobutyric acid-CONHC₂H₅), Calpain Inhibitor XI (Z-L- α-aminobutyric acid -CONH(CH₂)₃-morpholine), and Calpain Inhibitor XII (Z-L-Norvaline-CONH-CH₂-2-Pyridyl).

### 10. MEK Inhibitors

In certain embodiments, the at least one apoptosis inhibitor is a MEK1 or MEK2 inhibitor. Inhibitors of MEK1 and MEK2 are known (e.g., see US Patent Nos. 6,310,060; 6,440,966; 6,638,945; 7,001,905; 7,169,816; 7,745,663; 7,803,839; 7,897,624; 8,394,822, 8492,427 and 8,642,584), and commercially available.

Exemplary MEK inhibitors include, but are not limited to, PD0325901, N-[(2R)-2,3-dihydroxypropoxy]-3,4-difluoro-2-[(2-fluoro-4-iodophenyl)amino]-benzamide; MEK162, (5-[(4-bromo-2-fluorophenyl)amino]-4-fluoro-N-(2-hydroxyethoxy)-1-methyl-1H-benzimidazole-6-carboxamide), PD184352 (2-(2-chloro-4-iodophenylamino)-N-(cyclopropylmethoxy)-3,4-difluorobenzamide), pimasertib ((S)-N-(2,3-dihydroxypropyl)-3-(2-fluoro-4-iodophenylamino)isonicotinamide), selumetinib (6-(4-bromo-2-chlorophenylamino)-7-fluoro-N-(2-hydroxyethoxy)-3-methyl-3H-benzo[d]imidazole-5-carboxamide), trametinib(N-(3-(3-cyclopropyl-5-(2-fluoro-4-iodophenylamino)-6,8-dimethyl-2,4,7-trioxo-3,4,6,7-tetrahydropyrido[4,3-d]pyrimidin-1(2H)-yl)phenyl)acetamide), PD98059 (2-(2-amino-3-methoxyphenyl)-4H-chromen-4-one), U0126-EtOH ((2Z,3Z)-2,3-bis(amino(2-aminophenylthio)methylene)succinonitrile,ethanol).

In certain embodiments for substantially dry storage of cells for a period of greater than 24 hours, e.g., 48 or 120 hrs, PD0325901 may be added to the predehydration and/or rehydration formulation at a concentration range of about 1 nM to about 1.0 µM, preferably between about 10 nM and 500 nM, more preferably about 20 nM and 250 nM, and even more preferred about 50 nM.

### 11. PI3K Pathway Inhibitors

In certain other embodiments, the at least one apoptosis inhibitor is a PI3K inhibitor.

Exemplary PI3K inhibitors for use in the formulations, compositions and methods described herein include, but are not limited to, dactolisib (2-methyl-2-[4-(3-methyl-2-oxo-8-quinolin-3-ylimidazo[4,5-c]quinolin-1-yl)phenyl]propanenitrile), GDC-0941 (2-(1H-indazol-4-yl)-6-[[4-(methylsulfonyl)-1-piperazinyl]methyl]-4-(4-morpholinyl)thieno[3,2-d]pyrimidine), LY294002 (2-(4-morpholinyl)-8-phenyl-4H-1-benzopyran-4-one), idealalisib (5-fluoro-3-phenyl-2-[(1*S*)-1-(7*H*-purin-6-ylamino)propyl]-4(3*H*)-quinazolinone), burparlisib (5-(2,6-dimorpholinopyrimidin-4-yl)-4-(trifluoromethyl)pyridin-2-amine), GDC-0032 (4-[5,6-dihydro-2-[3-methyl-1-(1-methylethyl)-1H-1,2,4-triazol-5-yl]imidazo[1,2-d][1,4]benzoxazepin-9-yl]-α,α-dimethyl-1H-Pyrazole-1-acetamide), PI-103 (3-(4-(4-morpholinyl)pyrido[3',2':4,5]furo[3,2-d]pyrimidin-2-yl)phenol), NU7441 (8-(4-dibenzothienyl)-2-(4-morpholinyl)-4H-1-Benzopyran-4-one), GSK2636771 (2-methyl-1-[[2-methyl-3-(trifluoromethyl)phenyl]methyl]-6-(4-morpholinyl)-1H-benzimidazole-4-carboxylic acid), IPI-145 (8-chloro-2-phenyl-3-[(1S)-1-(9H-purin-6-ylamino)ethyl]-1-(2H)-isoquinolinone), XL147 (N-(3-(benzo[c][1,2,5]thiadiazol-5-ylamino)quinoxalin-2-yl)-4-methylbenzenesulfonamide), TGX-221 (7-methyl-2-(4-morpholinyl)-9-[1-(phenylamino)ethyl]-4H-pyrido[1,2-a]pyrimidin-4-one), PIK-90 (N-(7,8-dimethoxy-2,3-dihydro-imidazo[1,2-c]quinazolin-5-yl)-nicotinamide), wortmannin (11-(acetyloxy)-1,6b,7,8,9a,10,11,11b-octahydro-1-(methoxymethyl)-9a,11b-dimethyl-, (1S,6bR,9aS,11R,11bR)-3H-fluoro[4,3,2-de]indeno[4,5-h]-2-benzopyran-3,6,9-trione), VS-5584 (5-[8-methyl-9-(1-methylethyl)-2-(4-morpholinyl)-9H-purin-6-yl]-2-pyrimidinamine), and TG-100703 (3-(2,4-diamino-6-pteridinyl)-phenol).

In certain embodiments for substantially dry storage of cells for a period of greater than 24 hours, e.g., 48 or 120 hrs, LY294002 may be added to the predehydration and/or rehydration formulation at a concentration range of about 10 nM to about 2.0 µM, preferably between about 20 nM and 1.0 µM, more preferably about 50 nM and 500 nM, and even more preferred about 120 nM.

### ER CHAPERONE INDUCERS

In certain aspects, the predehydration formulation, dehydration and/or the rehydration formulation comprises at least one apoptosis inhibitor and an ER chaperone inducer. Suitable ER chaperone inducers for use in the formulations, compositions and methods described herein include, but are not limited to, BIX, valproate and lithium.

### 1. BIX

BiP inducer X (BIX) was identified in a screen for compounds that induce GRP78/BiP expression. BIX preferentially induced BiP with slight inductions of GRP94 (94 kDa glucose-regulated protein), calreticulin, and C/EBP homologous protein. The induction of BiP mRNA by BIX was mediated by activation of ER stress response elements upstream of the BiP gene, through the ATF6 (activating transcription factor 6) pathway.

### 2. Valproate

Valproic acid (2-propylpentanoic acid) has been approved for the treatment of epilepsy, bipolar mania and migraine prophylaxis. Valproic acid is a liquid at room temperature, but it can be reacted with a base such as sodium hydroxide to form the salt sodium valproate, which is a solid. The mechanism of action of valproate is not fully understood but it has been shown to inhibit CYP2C9, glucuronyl transferase, and epoxide hydrolase and leads to increased levels of gamma-aminobutyric acid (GABA) in the brain. The administration of valproate increases the expression of GRP78/BiP thereby stabilizing the three proximal transmembrane sensors, PERK, IRE1 and ATF6, in the favored inactivated state.

### 3. Lithium

The element lithium is used for treating various mood disorders. The administration of lithium increases the expression of GRP78/BiP thereby stabilizing the three proximal transmembrane sensors, PERK, IRE1 and ATF6, in the desired inactivated state.

### AUTOPHAGY INDUCERS

In certain other aspects, the predehydration formulation, dehydration and/or the rehydration formulation comprises at least one apoptosis inhibitor and an autophagy inducer. Autophagy inducers are series of diverse compounds that beneficially promote the lysosomal degradation of undesired or misfolded proteins thereby elevating the effect ofUPR on the cell. While not being bound to any theory, it is believed that the combination of the apoptosis inhibitor and autophagy inducer block the ER stress pathway while further promoting degradation of misfolded proteins that may arise as a result of dehydration to assist in the rehydrating the cell in a manner retain at least one functional property as the cells prior to dehydration.

Exemplary autophagy inducers include, but are not limited to, fluspirilene, trifluoperazine, pimozide, nicardipine, niguldipine, loperamide, amiodarone, rapamycin, resveratrol and SMERs.

In certain embodiments for substantially dry storage of cells for a period of greater than 24 hours, e.g., 48 or 120 hrs, rapamycin may be added to the predehydration and/or rehydration formulation at a concentration range of about 1 nM to about 1.0 µM, preferably between about 20 nM and 200 nM, more preferably about 20 nM and 80 nM, and even more preferred about 20 nM.

### SURVIVAL PROTEINS

In another aspect, the predehydration formulation, dehydration and/or the rehydration formulation comprise a survival protein. An exemplary survival protein is Bcl-xL.

Bcl-xL is a member of the BCL-2 family and is a transmembrane protein located in the mitochondria. Bcl is reported to exist in two forms, the long form Bcl-xL and Bcl-xS, a shorter splice variant form. Bcl-xL functions at the level of intrinsic apoptotic pathway, while extrinsic pathway (Fas/TNF death receptors) directly leads to caspase activation preventing the release of mitochondrial contents such as cytochrome c, which would lead to caspase activation. It is a well-established concept in the field of apoptosis that relative amounts of pro- and anti-survival Bcl-2 family of proteins define whether the cell will undergo cell death

In certain embodiments, Bcl-xL is delivered to the cells using liposome formulations to ensure adequate intracellular uptake of Bcl-xL.

### PREDEHYDRATION FORMULATIONS

In certain aspects, the compositions and methods for substantially dry storage of a cell include an additional step of prior to dehydration the cell is treated with a predehydration formulation. In one embodiment, the predehydration formulation comprises at least one apoptosis inhibitor, preferably a reversible apoptosis inhibitor. In another embodiment, the predehydration formulation comprises at least one apoptosis inhibitor and at least one ER chaperone inducer, at least one autophagy inducer or at least one survival protein.

In certain embodiments, the least one apoptosis inhibitor in the predehydration formulation is selected from the group consisting of a PERK-eIF2-α inhibitor, an ASK1 inhibitor, a NRF2-KEAP1 inhibitor, a JNK inhibitor, a p38 MAP kinase inhibitor, an IRE1 inhibitor, a GSK3 inhibitor, a MEK inhibitor, aPI3K pathway inhibitor, a calpain inhibitor, and a caspase-1 inhibitor.

In one embodiment, the least one apoptosis inhibitor in the predehydration formulation is a PERK-eIF2-α inhibitor. In certain embodiments, the PERK-eIF2-α inhibitor is selected from the group consisting of salubrinal, Sal-003 (3-phenyl-N-[2,2,2-trichloro-1-[(4-chlorophenyl)carbamothioylamino]ethyl]prop-2-enamide), GSK 2606414 (7-methyl-5-(1-{[3-(trifluoromethyl)phenyl]acetyl}-2,3-dihydro-1-H-indol-5-yl)7-H-pyrrolo[2,3d]pyrimidin-4-amine), GSK 2656157 (1-(5-(4-amino-7-methyl-7H-pyrrolo[2,3-d]pyrimidin-5-yl)-4-fluoroindolin-1-yl)-2-(6-methylpyridin-2-yl)ethanone) and ISRIB (trans-N,N'-(cyclohexane-1,4-diyl)bis(2-(4-chlorophenoxy)acetamide). In certain other embodiments, the PERK-eIF2-α inhibitor is salubrinal.

In another embodiment, the least one apoptosis inhibitor in the predehydration formulation is an ASK1 inhibitor, preferably NDQI-1 or MLS-0315763.

In yet another embodiment, the least one apoptosis inhibitor in the predehydration formulation is a NRF2-KEAP1 inhibitor. In certain embodiments, the NRF2-KEAP1 inhibitor is selected from the group consisting of carnosic acid, tri-terpenoids, sulphoraphane, and tert-butylhydroquinone.

In still another embodiment, the least one apoptosis inhibitor in the predehydration formulation is a GSK3 inhibitor. In certain embodiments, the GSK3 inhibitor is selected from the group consisting of CHIR98014 (N6-[2-[[4-(2,4-dichlorophenyl)-5-(1H-imidazol-2-yl)-2-pyrimidinyl]amino]ethyl]-3-nitro-2,6-pyridinediamine), valproate, CT 99021 and CT 20026.

In a further embodiment, the least one apoptosis inhibitor in the predehydration formulation is a MEK inhibitor. In certain embodiments, the MEK inhibitor is selected from the group consisting of PD0325901, N-[(2R)-2,3-dihydroxypropoxy]-3,4-difluoro-2-[(2-fluoro-4-iodophenyl)amino]-benzamide; MEK162, (5-[(4-bromo-2-fluorophenyl)amino]-4-fluoro-N-(2-hydroxyethoxy)-1-methyl-1H-benzimidazole-6-carboxamide), PD184352 (2-(2-chloro-4-iodophenylamino)-N-(cyclopropylmethoxy)-3,4-difluorobenzamide), pimasertib ((S)-N-(2,3-dihydroxypropyl)-3-(2-fluoro-4-iodophenylamino)isonicotinamide), selumetinib (6-(4-bromo-2-chlorophenylamino)-7-fluoro-N-(2-hydroxyethoxy)-3-methyl-3H-benzo[d]imidazole-5-carboxamide), trametinib (N-(3-(3-cyclopropyl-5-(2-fluoro-4-iodophenylamino)-6,8-dimethyl-2,4,7-trioxo-3,4,6,7-tetrahydropyrido[4,3-d]pyrimidin-1(2H)-yl)phenyl)acetamide), PD98059 (2-(2-amino-3-methoxyphenyl)-4H-chromen-4-one), and U0126-EtOH ((2Z,3Z)-2,3-bis(amino(2-aminophenylthio)methylene)succinonitrile,ethanol).

In still another embodiment, the least one apoptosis inhibitor in the predehydration formulation is a JNK inhibitor. In certain embodiments, the JNK inhibitor is selected from the group consisting of SP600125 (anthra[1-9-cd]pyrazol-6(2H)-one), JNK-IN-8 (3-[[4-(dimethylamino)-1-oxo-2-buten-1-yl]amino]-N-[3-methyl-4-[[4-(3-pyridinyl)-2-pyrimidinyl]amino]phenyl]-benzamide); JNK-Inhibitor IX (N-(3-cyano-4,5,6,7-tetrahydrobenzo[b]thien-2-yl)- 1-naphthalenecarboxamide).

In another embodiment, the least one apoptosis inhibitor in the predehydration formulation is a JNK inhibitor and a p38 MAP kinase inhibitor. In certain embodiments, the p38 MAP kinase inhibitor is selected from the group consisting of SB203580 (4-(4-(4-fluorophenyl)-2-(4-(methylsulfinyl)phenyl)-1H-imidazol-5-yl)pyridine), LY2228820 (5-(2-tert-butyl-4-(4-fhiorophenyl)-1H-imidazol-5-yl)-3-neopentyl-3H-imidazo[4,5-b]pyridin-2-amine dimethanesulfonate), PD169316 (4-(4-fluorophenyl)-2-(4-nitrophenyl)-5-(4-pyridyl)-1H-imidazole), PH-797804 (3-(4-(2,4-difluorobenzyloxy)-3-bromo-6-methyl-2-oxopyridin-1(2H)-yl)-N,4-dimethylbenzamide), SB202190 (4-(4-(4-fluorophenyl)-5-(pyridin-4-yl)-1H-imidazol-2-yl)phenol), BIRB 796 (Doramapimod; 1-(3-tert-butyl-1-p-tolyl-1H-pyrazol-5-yl)-3-(4-(2-morpholinoethoxy)naphthalen-1-yl)urea), VX-702 (1-(5-carbamoyl-6-(2,4-difluorophenyl)pyridin-2-yl)-1-(2,6-difluorophenyl)urea), and TAK-715 (N-[4-[2-ethyl-4-(3-methylphenyl)-5-thiazolyl]-2-pyridinyl]-benzamide.

In a further embodiment, the least one apoptosis inhibitor in the predehydration formulation is a PI3K inhibitor. In certain embodiments, the PI3K inhibitor is selected from the group consisting of dactolisib (2-methyl-2-[4-(3-methyl-2-oxo-8-quinolin-3-ylimidazo[4,5-c]quinolin-1-yl)phenyl]propanenitrile), GDC-0941 (2-(1H-indazol-4-yl)-6-[[4-(methylsulfonyl)-1-piperazinyl]methyl]-4-(4-morpholinyl)thieno[3,2-d]pyrimidine), LY294002 (2-(4-morpholinyl)-8-phenyl-4H-1-benzopyran-4-one), idealalisib (5-fluoro-3-phenyl-2-[(1*S*)-1-(7*H-*purin-6-ylamino)propyl]-4(3*H*)-quinazolinone), burparlisib (5-(2,6-dimorpholinopyrimidin-4-yl)-4-(trifluoromethyl)pyridin-2-amine), GDC-0032 (4-[5,6-dihydro-2-[3-methyl-1-(1-methylethyl)-1H-1,2,4-triazol-5-yl]imidazo[1,2-d][1,4]benzoxazepin-9-yl]-α,α-dimethyl-1H-pyrazole-1-acetamide), PI-103 (3-(4-(4-morpholinyl)pyrido[3',2':4,5]furo[3,2-d]pyrimidin-2-yl)phenol), NU7441 (8-(4-dibenzothienyl)-2-(4-morpholinyl)- 4H-1-benzopyran-4-one), GSK2636771 (2-methyl-1-[[2-methyl-3-(trifluoromethyl)phenyl]methyl]-6-(4-morpholinyl)-1H-benzimidazole-4-carboxylic acid), IPI-145 (8-chloro-2-phenyl-3-[(1S)-1-(9H-purin-6-ylamino)ethyl]-1-(2H)-isoquinolinone), XL147 (N-(3-(benzo[c][1,2,5]thiadiazol-5-ylamino)quinoxalin-2-yl)-4-methylbenzenesulfonamide), TGX-221 (7-methyl-2-(4-morpholinyl)-9-[1-(phenylamino)ethyl]-4H-pyrido[1,2-a]pyrimidin-4-one), PIK-90 (N-(7,8-dimethoxy-2,3-dihydro-imidazo[1,2-c]quinazolin-5-yl)-nicotinamide), wortmannin (11-(acetyloxy)-1,6b,7,8,9a,10,11,11b-octahydro-1-(methoxymethyl)-9a,11b-dimethyl-, (1S,6bR,9aS,11R,11bR)-3H-fluoro[4,3,2-de]indeno[4,5-h]-2-benzopyran-3,6,9-trione), VS-5584 (5-[8-methyl-9-(1-methylethyl)-2-(4-morpholinyl)-9H-purin-6-yl]-2-pyrimidinamine), and TG-100703 (3-(2,4-diamino-6-pteridinyl)-phenol).

In one embodiment, the least one apoptosis inhibitor in the predehydration formulation is an IRE-1 inhibitor. In certain embodiments, the IRE-1 inhibitor is selected from the group consisting of IRE1 Inhibitor I (N-[(2-hydroxynaphthalen-1-yl)methylidene]thiophene-2-sulfonamide), IRE1 Inhibitor II (3'-formyl-4'-hydroxy-5'-methoxybiphenyl-3-carboxamide), and IRE1 Inhibitor III (8-formyl-7-hydroxy-4-methylcoumarin, 7-hydroxy-4-methyl-2-oxo-2H-chromene-8-carbaldehyde).

In one embodiment, the least one apoptosis inhibitor in the predehydration formulation is a calpain inhibitor. In certain embodiments, the calpain inhibitor is selected from the group consisting of Calpain Inhibitor I (N-Acetyl-Leu-Leu-Norleucine-CHO), Calpain Inhibitor II (N-Acetyl-Leu-Leu-Met), Calpain Inhibitor III (Z-Val-Phe-CHO), Calpain Inhibitor IV (Z-Leu-Leu-Tyr-CH₂F), Calpain Inhibitor V (Morpholinoureidyl;-Val-homophenylalanine-CH₂F), Calpain Inhibitor VI (4-Fluorophenylsulfonyl-Val-Leu-CHO), Calpain Inhibitor X (Z-Leu-α-aminobutyric acid-CONHC₂H₅), Calpain Inhibitor XI (Z-L- α-aminobutyric acid -CONH(CH₂)₃-morpholine), and Calpain Inhibitor XII (Z-L-Norvaline-CONH-CH₂-2-Pyridyl).

In one embodiment, the least one apoptosis inhibitor in the predehydration formulation is a casapase-1 inhibitor. In certain embodiments, the caspase-1 inhibitor is selected from the group consisting of Caspase-1 Inhibitor II (Ac-YVAD-chloromethyl ketone), N-(2-Quinolyl)valyl-aspartyl-(2,6-difluorophenoxy)methyl ketone (in which the aspartyl residue is a- methylated or non-a-methylated), VX-765 ((S)-1-((S)-2-(4-amino-3-chlorobenzamido)-3,3-dimethylbutanoyl)-N-((2R,3S)-2-ethoxy-5-oxo-tetrahydrofuran-3-yl)pyrrolidine-2-carboxamide) and ZVAD-fluoromethyl ketone.

In another aspect, the predehydration formulation comprises at least one apoptosis inhibitor and at least one ER chaperone inducer. In certain embodiments, the ER chaperone inducer is selected from the group consisting of BIX, valproate and lithium.

In another aspect, the predehydration formulation comprises at least one apoptosis inhibitor and at least one autophagy inducer. In certain embodiments, the autophagy inducer is selected from the group consisting of fluspirilene, trifluoperazine, pimozide, nicardipine, niguldipine, loperamide, amiodarone, rapamycin, resveratrol and SMERs.

In another aspect, the predehydration formulation comprises at least one apoptosis inhibitor and at least one survival protein. In one embodiment, the survival protein is Bcl-xL.

### REHYDRATION FORMULATIONS

In certain other aspects, the compositions and methods for substantially dry storage of a cell further comprises rehydrating the substantially dry stored cell using a rehydration formulation. In one embodiment, the rehydration formulation comprises at least one apoptosis inhibitor, preferably a reversible apoptosis inhibitor. In another embodiment, the rehydration formulation comprises at least one apoptosis inhibitor and at least one ER chaperone inducer.

In certain embodiments, the least one apoptosis inhibitor in the rehydration formulation is selected from the group consisting of a PERK-eIF2-α inhibitor, an ASK1 inhibitor, a NRF2-KEAP1 inhibitor, a JNK inhibitor, a p38 MAP kinase inhibitor, an IRE1 inhibitor, a GSK3 inhibitor, a MEK inhibitor, aPI3K pathway inhibitor, a calpain inhibitor, and a caspase-1 inhibitor.

In one embodiment, the least one apoptosis inhibitor in the rehydration formulation is a PERK-eIF2-α inhibitor. In certain embodiments, the PERK-eIF2-α inhibitor is selected from the group consisting of salubrinal, Sal-003 (3-phenyl-N-[2,2,2-trichloro-1-[(4-chlorophenyl)carbamothioylamino]ethyl]prop-2-enamide), GSK 2606414 (7-methyl-5-(1-{[3-(trifluoromethyl)phenyl]acetyl}-2,3-dihydro-1-H-indol-5-yl)7-H-pyrrolo[2,3d]pyrimidin-4-amine), GSK 2656157 (1-(5-(4-amino-7-methyl-7H-pyrrolo[2,3-d]pyrimidin-5-yl)-4-fluoroindolin-1-yl)-2-(6-methylpyridin-2-yl)ethanone) and ISRIB (trans-N,N'-(cyclohexane-1,4-diyl)bis(2-(4-chlorophenoxy)acetamide). In certain other embodiments, the PERK-eIF2-α inhibitor is salubrinal.

In another embodiment, the least one apoptosis inhibitor in the rehydration formulation is an ASK1 inhibitor, preferably NDQI-1 or MLS-0315763.

In yet another embodiment, the least one apoptosis inhibitor in the rehydration formulation is a NRF2-KEAP1 inhibitor. In certain embodiments, the NRF2-KEAP1 inhibitor is selected from the group consisting of carnosic acid, tri-terpenoids, sulphoraphane, and tert-butylhydroquinone.

In still another embodiment, the least one apoptosis inhibitor in the rehydration formulation is a GSK3 inhibitor. In certain embodiments, the GSK3 inhibitor is selected from the group consisting of CHIR98014 (N6-[2-[[4-(2,4-dichlorophenyl)-5-(1H-imidazol-2-yl)-2-pyrimidinyl]amino]ethyl]-3-nitro-2,6-pyridinediamine), valproate, CT 99021 and CT 20026.

In a further embodiment, the least one apoptosis inhibitor in the rehydration formulation is a MEK inhibitor. In certain embodiments, the MEK inhibitor is selected from the group consisting of PD0325901, N-[(2R)-2,3-dihydroxypropoxy]-3,4-difluoro-2-[(2-fluoro-4-iodophenyl)amino]-benzamide; MEK162, (5-[(4-bromo-2-fluorophenyl)amino]-4-fluoro-N-(2-hydroxyethoxy)-1-methyl-1H-benzimidazole-6-carboxamide), PD184352 (2-(2-chloro-4-iodophenylamino)-N-(cyclopropylmethoxy)-3,4-difluorobenzamide), pimasertib ((S)-N-(2,3-dihydroxypropyl)-3-(2-fluoro-4-iodophenylamino)isonicotinamide), selumetinib (6-(4-bromo-2-chlorophenylamino)-7-fluoro-N-(2-hydroxyethoxy)-3-methyl-3H-benzo[d]imidazole-5-carboxamide), trametinib (N-(3-(3-cyclopropyl-5-(2-fluoro-4-iodophenylamino)-6,8-dimethyl-2,4,7-trioxo-3,4,6,7-tetrahydropyrido[4,3-d]pyrimidin-1(2H)-yl)phenyl)acetamide), PD98059 (2-(2-amino-3-methoxyphenyl)-4H-chromen-4-one), and U0126-EtOH ((2Z,3Z)-2,3-bis(amino(2 -aminophenylthio)methylene)succinonitrile,ethanol).

In still another embodiment, the least one apoptosis inhibitor in the rehydration formulation is a JNK inhibitor. In certain embodiments, the JNK inhibitor is selected from the group consisting of SP600125 (anthra[1-9-cd]pyrazol-6(2H)-one), JNK-IN-8 (3-[[4-(dimethylamino)-1-oxo-2-buten-1-yl]amino]-N-[3-methyl-4-[[4-(3-pyridinyl)-2-pyrimidinyl]amino]phenyl]-benzamide); JNK-Inhibitor IX (N-(3-cyano-4,5,6,7-tetrahydrobenzo[b]thien-2-yl)-1-naphthalenecarboxamide).

In another embodiment, the least one apoptosis inhibitor in the rehydration formulation is a JNK inhibitor and a p38 MAP kinase inhibitor. In certain embodiments, the p38 MAP kinase inhibitor is selected from the group consisting of SB203580 (4-(4-(4-fluorophenyl)-2-(4-(methylsulfinyl)phenyl)-1H-imidazol-5-yl)pyridine), LY2228820 (5-(2-tert-butyl-4-(4-fluorophenyl)-1H-imidazol-5-yl)-3-neopentyl-3H-imidazo[4,5-b]pyridin-2-amine dimethanesulfonate), PD169316 (4-(4-fluorophenyl)-2-(4-nitrophenyl)-5-(4-pyridyl)-1H-imidazole), PH-797804 (3-(4-(2,4-difluorobenzyloxy)-3-bromo-6-methyl-2-oxopyridin-1(2H)-yl)-N,4-dimethylbenzamide), SB202190 (4-(4-(4-fluorophenyl)-5-(pyridin-4-yl)-1H-imidazol-2-yl)phenol), BIRB 796 (Doramapimod; 1-(3-tert-butyl-1-p-tolyl-1H-pyrazol-5-yl)-3-(4-(2-morpholinoethoxy)naphthalen-1-yl)urea), VX-702 (1-(5-carbamoyl-6-(2,4-difluorophenyl)pyridin-2-yl)-1-(2,6-difluorophenyl)urea), and TAK-715 (N-[4-[2-ethyl-4-(3-methylphenyl)-5-thiazolyl]-2-pyridinyl]-benzamide.

In a further embodiment, the least one apoptosis inhibitor in the rehydration formulation is a PI3K inhibitor. In certain embodiments, the PI3K inhibitor is selected from the group consisting of dactolisib (2-methyl-2-[4-(3-methyl-2-oxo-8-quinolin-3-ylimidazo[4,5-c]quinolin-1-yl)phenyl]propanenitrile), GDC-0941 (2-(1H-Indazol-4-yl)-6-[[4-(methylsulfonyl)-1-piperazinyl]methyl]-4-(4-morpholinyl)thieno[3,2-d]pyrimidine), LY294002 (2-(4-morpholinyl)-8-phenyl-4H-1-benzopyran-4-one), idealalisib (5-fluoro-3-phenyl-2-[(1*S*)-1-(7*H*-purin-6-ylamino)propyl]-4(3*H*)-quinazolinone), burparlisib (5-(2,6-dimorpholinopyrimidin-4-yl)-4-(trifluoromethyl)pyridin-2-amine), GDC-0032 (4-[5,6-dihydro-2-[3-methyl-1-(1-methylethyl)-1H-1,2,4-triazol-5-yl]imidazo[1,2-d][1,4]benzoxazepin-9-yl]-α,α-dimethyl-1H-pyrazole-1-acetamide), PI-103 (3-(4-(4-morpholinyl)pyrido[3',2':4,5]furo[3,2-d]pyrimidin-2-yl)phenol), NU7441 (8-(4-dibenzothienyl)-2-(4-morpholinyl)- 4H-1-benzopyran-4-one), GSK2636771 (2-methyl-1-[[2-methyl-3-(trifluoromethyl)phenyl]methyl]-6-(4-morpholinyl)-1H-benzimidazole-4-carboxylic acid), IPI-145 (8-chloro-2-phenyl-3-[(1S)-1-(9H-purin-6-ylamino)ethyl]-1-(2H)-isoquinolinone), XL147 (N-(3-(benzo[c][1,2,5]thiadiazol-5-ylamino)quinoxalin-2-yl)-4-methylbenzenesulfonamide), TGX-221 (7-methyl-2-(4-morpholinyl)-9-[1-(phenylamino)ethyl]-4H-pyrido[1,2-a]pyrimidin-4-one), PIK-90 (N-(7,8-Dimethoxy-2,3-dihydro-imidazo[1,2-c]quinazolin-5-yl)-nicotinamide), wortmannin (11-(acetyloxy)-1,6b,7,8,9a,10,11,11b-octahydro-1-(methoxymethyl)-9a,11b-dimethyl-, (1S,6bR,9aS,11R,11bR)- 3H-Fluoro[4,3,2-de]indeno[4,5-h]-2-benzopyran-3,6,9-trione), VS-5584 (5-[8-methyl-9-(1-methylethyl)-2-(4-morpholinyl)-9H-purin-6-yl]-2-pyrimidinamine), and TG-100703 (3-(2,4-diamino-6-pteridinyl)-phenol).

In one embodiment, the least one apoptosis inhibitor in the rehydration formulation is an IRE-1 inhibitor. In certain embodiments, the IRE-1 inhibitor is selected from the group consisting of IRE1 Inhibitor I (N-[(2-hydroxynaphthalen-1-yl)methylidene]thiophene-2-sulfonamide), IRE1 Inhibitor II (3'-formyl-4'-hydroxy-5'-methoxybiphenyl-3-carboxamide), and IRE1 Inhibitor III (8-formyl-7-hydroxy-4-methylcoumarin, 7-hydroxy-4-methyl-2-oxo-2H-chromene-8-carbaldehyde).

In one embodiment, the least one apoptosis inhibitor in the rehydration formulation is a calpain inhibitor. In certain embodiments, the calpain inhibitor is selected from the group consisting of Calpain Inhibitor I (N-Acetyl-Leu-Leu-Norleucine-CHO), Calpain Inhibitor II (N-Acetyl-Leu-Leu-Met), Calpain Inhibitor III (Z-Val-Phe-CHO), Calpain Inhibitor IV (Z-Leu-Leu-Tyr-CH₂F), Calpain Inhibitor V (Morpholinoureidyl;-Val-homophenylalanine-CH₂F), Calpain Inhibitor VI (4-Fluorophenylsulfonyl-Val-Leu-CHO), Calpain Inhibitor X (Z-Leu-α-aminobutyric acid-CONHC₂H₅), Calpain Inhibitor XI (Z-L- α-aminobutyric acid -CONH(CH₂)₃-morpholine), and Calpain Inhibitor XII (Z-L-Norvaline-CONH-CH₂-2-Pyridyl).

In one embodiment, the least one apoptosis inhibitor in the rehydration formulation is a casapase-1 inhibitor. In certain embodiments, the caspase-1 inhibitor is selected from the group consisting of Caspase-1 Inhibitor II (Ac-YVAD-chloromethyl ketone), N-(2-Quinolyl)valyl-aspartyl-(2,6-difluorophenoxy)methyl ketone (in which the aspartyl residue is a- methylated or non-a-methylated), VX-765 ((S)-1-((S)-2-(4-amino-3-chlorobenzamido)-3,3-dimethylbutanoyl)-N-((2R,3S)-2-ethoxy-5-oxo-tetrahydrofuran-3-yl)pyrrolidine-2-carboxamide) and ZVAD-fluoromethyl ketone.

In another aspect, the rehydration formulation comprises at least one apoptosis inhibitor and at least one ER chaperone inducer. In certain embodiments, the ER chaperone inducer is selected from the group consisting of BIX, valproate and lithium.

In another aspect, the rehydration formulation comprises at least one apoptosis inhibitor and at least one autophagy inducer. In certain embodiments, the autophagy inducer is selected from the group consisting of fluspirilene, trifluoperazine, pimozide, nicardipine, niguldipine, loperamide, amiodarone, rapamycin, resveratrol and SMERs.

In another aspect, the rehydration formulation comprises at least one apoptosis inhibitor and at least one survival protein. In one embodiment, the survival protein is Bcl-xL.

### METHODS

In another aspect of the invention, methods are provided for substantially dry storage of one or more cell at ambient temperatures in the absence of refrigeration or lypholization, comprising incubating the one or more cell with a dehydration formulation comprising a dry storage stabilizer and dehydrating the one or more pretreated cell in the presence of a dehydration formulation to generate one or more substantially dry stored cell. In certain embodiments, the dehydration formulation further comprises at least one apoptosis inhibitor and the method may further comprise rehydrating the one or more substantially dry stored cell using a rehydration buffer comprising at least one apoptosis inhibitor.

In another aspect of the invention, methods are provided for substantially dry storage of one or more cell at ambient temperatures in the absence of refrigeration or lypholization, comprising incubating the one or more cell with a predehydration formulation comprising an apoptosis inhibitor to generate one or more pretreated cell, removing the predehydration formulation; and dehydrating the one or more pretreated cell in the presence of a dehydration formulation to generate one or more substantially dry stored cell. In certain embodiments, the method may further comprise rehydrating the one or more substantially dry stored cell using a rehydration buffer comprising at least one apoptosis inhibitor.

A number of apoptosis inhibitors can have deleterious effects on cells at high concentrations or for prolonged exposure periods. Conversely, exposing the cells to the predehydration formulation or rehydration formulation for too short of a period or at too low of an apoptosis inhibitor concentration will not result in the desired additional treatment effect during dehydration. Thus, the methods for substantially dry storage of cells using a predehydration step and a rehydration step exposure times need to be properly controlled to achieve the desired inhibitory effect.

In certain embodiments, the least one apoptosis inhibitor used in the methods is selected from the group consisting of a PERK-eIF2-α inhibitor, an ASK1 inhibitor, a NRF2-KEAP1 inhibitor, a JNK inhibitor, a p38 MAP kinase inhibitor, an IRE1 inhibitor, a GSK3 inhibitor, a MEK inhibitor, aPI3K pathway inhibitor, a calpain inhibitor, and a caspase-1 inhibitor.

In one embodiment, the least one apoptosis inhibitor used in the methods is a PERK-eIF2-α inhibitor. In certain embodiments, the PERK-eIF2-α inhibitor is selected from the group consisting of salubrinal, Sal-003 (3-phenyl-N-[2,2,2-trichloro-1-[(4-chlorophenyl)carbamothioylamino]ethyl]prop-2-enamide), GSK 2606414 (7-methyl-5-(1-{[3-(trifluoromethyl)phenyl]acetyl}-2,3-dihydro-1-H-indol-5-yl)7-H-pyrrolo[2,3d]pyrimidin-4-amine), GSK 2656157 (1-(5-(4-amino-7-methyl-7H-pyrrolo[2,3-d]pyrimidin-5-yl)-4-fluoroindolin-1-yl)-2-(6-methylpyridin-2-yl)ethanone) and ISRIB (trans-N,N'-(cyclohexane-1,4-diyl)bis(2-(4-chlorophenoxy)acetamide). In certain other embodiments, the PERK-eIF2-α inhibitor is salubrinal.

In another embodiment, the least one apoptosis inhibitor used in the methods is an ASK1 inhibitor, preferably NDQI-1 or MLS-0315763.

In yet another embodiment, the least one apoptosis inhibitor used in the methods is a NRF2-KEAP1 inhibitor. In certain embodiments, the NRF2-KEAP1 inhibitor is selected from the group consisting of carnosic acid, tri-terpenoids, sulphoraphane, and tert-butylhydroquinone.

In still another embodiment, the least one apoptosis inhibitor used in the methods is a GSK3 inhibitor. In certain embodiments, the GSK3 inhibitor is selected from the group consisting of CHIR98014 (N6-[2-[[4-(2,4-dichlorophenyl)-5-(1H-imidazol-2-yl)-2-pyrimidinyl]amino]ethyl]-3-nitro-2,6-pyridinediamine), valproate, CT 99021 and CT 20026.

In a further embodiment, the least one apoptosis inhibitor used in the methods is a MEK inhibitor. In certain embodiments, the MEK inhibitor is selected from the group consisting of PD0325901, N-[(2R)-2,3-dihydroxypropoxy]-3,4-difluoro-2-[(2-fluoro-4-iodophenyl)amino]-benzamide; MEK162, (5-[(4-bromo-2-fluorophenyl)amino]-4-fluoro-N-(2-hydroxyethoxy)-1-methyl-1H-benzimidazole-6-carboxamide), PD184352 (2-(2-chloro-4-iodophenylamino)-N-(cyclopropylmethoxy)-3,4-difluorobenzamide), pimasertib ((S)-N-(2,3-dihydroxypropyl)-3-(2-fluoro-4-iodophenylamino)isonicotinamide), selumetinib (6-(4-bromo-2-chlorophenylamino)-7-fluoro-N-(2-hydroxyethoxy)-3-methyl-3H-benzo[d]imidazole-5-carboxamide), trametinib (N-(3-(3-cyclopropyl-5-(2-fluoro-4-iodophenylamino)-6,8-dimethyl-2,4,7-trioxo-3,4,6,7-tetrahydropyrido[4,3-d]pyrimidin-1(2H)-yl)phenyl)acetamide), PD98059 (2-(2-amino-3-methoxyphenyl)-4H-chromen-4-one), and U0126-EtOH ((2Z,3Z)-2,3-bis(amino(2-aminophenylthio)methylene)succinonitrile,ethanol).

In still another embodiment, the least one apoptosis inhibitor used in the methods is a JNK inhibitor. In certain embodiments, the JNK inhibitor is selected from the group consisting of SP600125 (anthra[1-9-cd]pyrazol-6(2H)-one), JNK-IN-8 (3-[[4-(dimethylamino)-1-oxo-2-buten-1-yl]amino]-N-[3-methyl-4-[[4-(3-pyridinyl)-2-pyrimidinyl]amino]phenyl]-benzamide); JNK-Inhibitor IX (N-(3-cyano-4,5,6,7-tetrahydrobenzo[b]thien-2-yl)- 1-naphthalenecarboxamide).

In another embodiment, the least one apoptosis inhibitor used in the methods is a JNK inhibitor and a p38 MAP kinase inhibitor. In certain embodiments, the p38 MAP kinase inhibitor is selected from the group consisting of SB203580 (4-(4-(4-fluorophenyl)-2-(4-(methylsulfinyl)phenyl)-1H-imidazol-5-yl)pyridine), LY2228820 (5-(2-tert-butyl-4-(4-fluorophenyl)-1H-imidazol-5-yl)-3-neopentyl-3H-imidazo[4,5-b]pyridin-2-amine dimethanesulfonate), PD169316 (4-(4-fluorophenyl)-2-(4-nitrophenyl)-5-(4-pyridyl)-1H-imidazole), PH-797804 (3-(4-(2,4-difluorobenzyloxy)-3-bromo-6-methyl-2-oxopyridin-1(2H)-yl)-N,4-dimethylbenzamide), SB202190 (4-(4-(4-fluorophenyl)-5-(pyridin-4-yl)-1H-imidazol-2-yl)phenol), BIRB 796 (Doramapimod; 1-(3-tert-butyl-1-p-tolyl-1H-pyrazol-5-yl)-3-(4-(2-morpholinoethoxy)naphthalen-1-yl)urea), VX-702 (1-(5-carbamoyl-6-(2,4-difluorophenyl)pyridin-2-yl)-1-(2,6-difluorophenyl)urea), and TAK-715 (N-[4-[2-ethyl-4-(3-methylphenyl)-5-thiazolyl]-2-pyridinyl]-benzamide.

In a further embodiment, the least one apoptosis inhibitor used in the methods is a PI3K inhibitor. In certain embodiments, the PI3K inhibitor is selected from the group consisting of dactolisib (2-methyl-2-[4-(3-methyl-2-oxo-8-quinolin-3-ylimidazo[4,5-c]quinolin-1-yl)phenyl]propanenitrile), GDC-0941 (2-(1H-Indazol-4-yl)-6-[[4-(methylsulfonyl)-1-piperazinyl]methyl]-4-(4-morpholinyl)thieno[3,2-d]pyrimidine), LY294002 (2-(4-morpholinyl)-8-phenyl-4H-1-benzopyran-4-one), idealalisib (5-Fluoro-3-phenyl-2-[(1*S*)-1-(7*H*-purin-6-ylamino)propyl]-4(3*H*)-quinazolinone), burparlisib (5-(2,6-dimorpholinopyrimidin-4-yl)-4-(trifluoromethyl)pyridin-2-amine), GDC-0032 (4-[5,6-dihydro-2-[3-methyl-1-(1-methylethyl)-1H-1,2,4-triazol-5-yl]imidazo[1,2-d][1,4]benzoxazepin-9-yl]-α,α-dimethyl-1H-pyrazole-1-acetamide), PI-103 (3-(4-(4-morpholinyl)pyrido[3',2':4,5]furo[3,2-d]pyrimidin-2-yl)phenol), NU7441 (8-(4-dibenzothienyl)-2-(4-morpholinyl)- 4H-1-benzopyran-4-one), GSK2636771 (2-methyl-1-[[2-methyl-3-(trifluoromethyl)phenyl]methyl]-6-(4-morpholinyl)-1H-benzimidazole-4-carboxylic acid), IPI-145 (8-chloro-2-phenyl-3-[(1S)-1-(9H-purin-6-ylamino)ethyl]-1-(2H)-isoquinolinone), XL147 (N-(3-(benzo[c][1,2,5]thiadiazol-5-ylamino)quinoxalin-2-yl)-4-methylbenzenesulfonamide), TGX-221 (7-methyl-2-(4-morpholinyl)-9-[1-(phenylamino)ethyl]-4H-pyrido[1,2-a]pyrimidin-4-one), PIK-90 (N-(7,8-dimethoxy-2,3-dihydro-imidazo[1,2-c]quinazolin-5-yl)-nicotinamide), wortmannin (11-(acetyloxy)-1,6b,7,8,9a,10,11,11b-octahydro-1-(methoxymethyl)-9a,11b-dimethyl-, (1S,6bR,9aS,11R,11bR)-3H-Fluoro[4,3,2-de]indeno[4,5-h]-2-benzopyran-3,6,9-trione), VS-5584 (5-[8-methyl-9-(1-methylethyl)-2-(4-morpholinyl)-9H-purin-6-yl]- 2-pyrimidinamine), and TG-100703 (3-(2,4-diamino-6-pteridinyl)-phenol).

In one embodiment, the least one apoptosis inhibitor used in the methods is an IRE-1 inhibitor. In certain embodiments, the IRE-1 inhibitor is selected from the group consisting of IRE1 Inhibitor I (N-[(2-Hydroxynaphthalen-1-yl)methylidene]thiophene-2-sulfonamide), IRE1 Inhibitor II (3'-formyl-4'-hydroxy-5'-methoxybiphenyl-3-carboxamide), and IRE1 Inhibitor III (8-formyl-7-hydroxy-4-methylcoumarin, 7-hydroxy-4-methyl-2-oxo-2H-chromene-8-carbaldehyde).

In one embodiment, the least one apoptosis inhibitor used in the methods is a calpain inhibitor. In certain embodiments, the calpain inhibitor is selected from the group consisting of Calpain Inhibitor I (N-Acetyl-Leu-Leu-Norleucine-CHO), Calpain Inhibitor II (N-Acetyl-Leu-Leu-Met), Calpain Inhibitor III (Z-Val-Phe-CHO), Calpain Inhibitor IV (Z-Leu-Leu-Tyr-CH₂F), Calpain Inhibitor V (Morpholinoureidyl;-Val-homophenylalanine-CH₂F), Calpain Inhibitor VI (4-Fluorophenylsulfonyl-Val-Leu-CHO), Calpain Inhibitor X (Z-Leu-α-aminobutyric acid-CONHC₂H₅), Calpain Inhibitor XI (Z-L- α-aminobutyric acid -CONH(CH₂)₃-morpholine), and Calpain Inhibitor XII (Z-L-Norvaline-CONH-CH₂-2-Pyridyl).

In one embodiment, the least one apoptosis inhibitor used in the methods is a casapase-1 inhibitor. In certain embodiments, the caspase-1 inhibitor is selected from the group consisting of Caspase-1 Inhibitor II (Ac-YVAD-chloromethyl ketone), N-(2-Quinolyl)valyl-aspartyl-(2,6-difluorophenoxy)methyl ketone (in which the aspartyl residue is a- methylated or non-a-methylated), VX-765 ((S)-1-((S)-2-(4-amino-3-chlorobenzamido)-3,3-dimethylbutanoyl)-N-((2R,3S)-2-ethoxy-5-oxo-tetrahydrofuran-3-yl)pyrrolidine-2-carboxamide) and ZVAD-fluoromethyl ketone.

In another aspect, the rehydration formulation used in the methods comprises at least one apoptosis inhibitor and at least one ER chaperone inducer. In certain embodiments, the ER chaperone inducer is selected from the group consisting of BIX, valproate and lithium.

In another aspect, the rehydration formulation used in the methods comprises at least one apoptosis inhibitor and at least one autophagy inducer. In certain embodiments, the autophagy inducer is selected from the group consisting of fluspirilene, trifluoperazine, pimozide, nicardipine, niguldipine, loperamide, amiodarone, rapamycin, resveratrol and SMERs.

In another aspect, the rehydration formulation used in the methods comprises at least one apoptosis inhibitor and at least one survival protein. In one embodiment, the survival protein is Bcl-xL.

In certain embodiments, the at least one apoptosis inhibitor is a reversible apoptosis inhibitor. In the methods, the reversible apoptosis inhibitor may be used to treat the cells during the predehydration and/or rehydration phases to load the cells with a protective amount of the apoptosis inhibitor, e.g., to block one or more ER stress pathway activation, such that upon removal of the predehydration and/or rehydration formulation and resuspending the cells the reversible inhibitor eventually is diluted from the cells to avoid prolonged exposure periods.

### KITS

In certain other aspects, kit are provided comprising a liquid dehydration formulation comprising a dry storage stabilizer, a sample container for placing one or more cell for substantially dry storage, and a packaging insert comprising directions for use for substantially dry storage of one or more cell using the liquid dehydration formulation. In certain embodiments, the dehydration formulation further comprises at least one apoptosis inhibitor and may further still comprise a rehydration buffer comprising at least one apoptosis inhibitor.

In certain other embodiments, the kits further comprising a solid support for immobilizing one or more cell prior to dehydration, a predehydration formulation comprising at least one apoptosis inhibitor, a dehydration formulation comprising at least one dry storage stabilizer for substantially dry storage of the one or more cell, and a packing insert comprising directions for immobilizing the one or more cell to the solid support and for substantially dry storage of one or more cell using the predehydration formulation and dehydration formulation. In yet another embodiment, the kits may further comprise a rehydration buffer comprising at least one apoptosis inhibitor.

### GENERAL PROTOCOL FOR SCREENING FORMULATIONS

The following protocol may be employed for analyzing and selecting predehydration, dehydration and/or rehydration formulations and combinations thereof for substantially dry storage of cells that retain at least one functional property for at least one hour post-rehydration. Briefly, cells are seeded in 96-well plates (20,000 cells/well) in DMEM medium or a predehydration formulation (e.g., DMEM medium + at least one apoptosis inhibitor) and incubated at 37°C for an hour to up to 24 hours. After incubation, the medium is aspirated from the cells and discarded, and 10 µl of dehydration formulation is added to each well. The open 96-well plate is incubated at 37°C for 75 minutes if drying a half-full 96-well plate or for 95 minutes if drying a full 96-well plate. The dried cells are stored at room temperature for 1 hour and rehydrated with variable amount of rehydration formulation comprising at least one apoptosis inhibitor or in complete DMEM medium. The rehydrated cells are incubated in a 37°C CO₂-regulated incubator for a period of 1 hour or 24 hours. At the designated time point, trypan blue is added to the rehydrated cells and the cells are counted using a cytometer. The percent cell survival is determined by dividing the number of trypan blue stained cells by the total number of cells to determine the fraction of non-viable cells, and then calculating the percent of surviving cells.

### ASSAYS FOR MEASURING FUNCTIONAL PROPERTIES OF CELLS

The substantially dry stored cells of the present invention retain upon rehydration at least one functional property of the cells prior to undergoing dehydration. The one functional property may selected from the group of a metabolic activity, cell viability, the ability to proliferate, differentiate, respond to signaling stimuli such that imparted by growth factors, and expression expected cell biomarkers such as RNA synthesis, protein, and or secretory functions. These functional properties may be detected or analyzed using any method, including the methods disclosed herein as well as other methods known to those skilled in the art. Exemplary methods for detecting at least one functional property of rehydrated dry stored cells are described below.

### 1. Cell Viability Assays

Cell viability may be measured using a Trypan Blue staining procedure. Trypan Blue is a dye with a negatively charged chromophore that does not interact with a cell unless its cellular membrane is damaged, and therefore viable cells exclude the dye, while damaged cells appear blue when examined under the microscope. Cell counting was performed in 9mm KOVA glass slides 10 with Grid Chambers (Hycor) in triplicate under a Leitz Fluovert microscope. The percentage of cell survival is reported relative to untreated control cells.

### 2. ATP Content Assays

ATP content may be determined using a Cell Titer-Glo Luminiscent Cell viability Assay (Promega) in accordance with the manufacturer's instructions. The addition of the Cell Titer-Glo Luminiscent reagent into the cells generates a luminescent signal proportional to the amount of intracellular ATP. The amount of ATP is directly proportional to the number of cells present in culture, and it is then a homogeneous method of determining the number of viable (metabolically active) cells in the rehydrated cell preparation.

### 3. Caspase Assays

The degree of cellular apoptosis may be measured using a Caspase-Glo 3/7 Assay (Promega) according to the manufacturer's instructions. Briefly, this assay provides a proluminescent caspase-3/7 substrate, which contains the tetrapeptide sequence DEVD. This substrate is cleaved to release aminoluciferin, a substrate of luciferase used in the production of light. The addition of the single Caspase-Glo 3/7 Reagent results in cell lysis, followed by caspase cleavage of the substrate and generation of a luminescent signal. The fold caspase activation was calculated as a ratio of activity in the test samples relative to untreated cells cultured under standard tissue culture conditions.

The following Examples are presented by way of illustration and not limitation.

### EXAMPLE 1

### EXEMPLARY FORMULATIONS MAINTAIN VIABLE CELLS AND PREVENT CELLULAR APOPTOSIS OF SUBSTANTIALLY DRY STORED CELLS FIVE DAYS POST-REHYDRATION

This example demonstrates that exemplary predehydration, dehydration and rehydration formulations described herein maintain cell viability and prevent cells from inducing apoptosis up to 5 days post-rehydration after substantially dry storage for 5 hours at ambient temperatures.

HeLa cells were substantially dry stored using predetermined predehydration formulations (SC1, salubrinal and SC3, MLS-0315763), then treated with a subset of the formulations shown in Table 1, and dehydrated in 96 well plates and stored at ambient temperature for a period of 5 hours. The cells were rehydrated using the rehydration formulations (RC1, salubrinal and RC3, MLS-0315763) and ATP content was measured by the addition of CellTiter-glo to the 96-well plate. After the cells had lysed, a 50 µl sample was transferred to a white 384-well plate for quantitation. The substantially dry stored cells were assayed for cell viability by measuring ATP luminescence.

Furthermore, those substantially dry stored cells evidencing positive ATP activity were assayed 5 days post rehydration for ATP content. As shown in Table 2, all of the non-formulation control cells were non-viable at Day 5 whereas a significant proportion of the cells substantially dry stored using the formulations and methods described herein remain viable, as high as 90% cell viability, showing stabilization of intact, metabolically-active cells.

The rehydrated cells also were analyzed to determine whether substantial dry storage for 5 days followed by 5 days of rehydration resulted in the induction of cellular apoptosis by measuring caspase activity. The 5 day rehydrated cells were exposed to Caspase-glo (Promega) to detect activity of caspase 3/7. Caspase activation was calculated as a ratio of activity in the test samples relative to untreated cells cultured under standard tissue culture conditions (Table 2). A result of one or below one is considered to be a results demonstrating that the absence of elevated caspase activity and that no apoptosis is observed.

**TABLE 2**

| EXEMPLARY FORMULATIONS MAINTAIN VIABLE CELLS AND PREVENT CELLULAR APOPTOSIS OF SUBSTANTIALLY DRY STORED CELLS FIVE DAYS POST-REHYDRATION | | |
|---|---|---|
| **Formulations** | **% Cell Viability** | **Fold Caspase 3/7 Activation** |
| NF Control | 0 | 0.25 |
| SC3 + MCS41 + RC3 | 88 | 0.7 |
| SC3 + MCS21 + RC3 | 90 | 0.7 |
| SC1 + MCS41 + RC1 | 79 | 1.0 |
| SC1 + MCS43 + RC1 | 78 | 0.8 |
| SC1 + MCS42 + RC1 | 60 | 0.5 |

As shown in Table 2, little to no detectable caspase activity observed over background values demonstrating that apoptosis was not induced after dry storage for 5 days followed by rehydration for a period of at least 5 days.

### EXAMPLE 2

### EXEMPLARY FORMULATIONS MAINTAIN VIABLE CELLS AFTER SUBSTANTIALLY DRY STORAGE AFTER SEVEN DAYS POST-REHYDRATION

This Example demonstrates that exemplary formulations described herein are capable of maintaining viable HeLa cells for a period of at least seven hours post-rehydration.

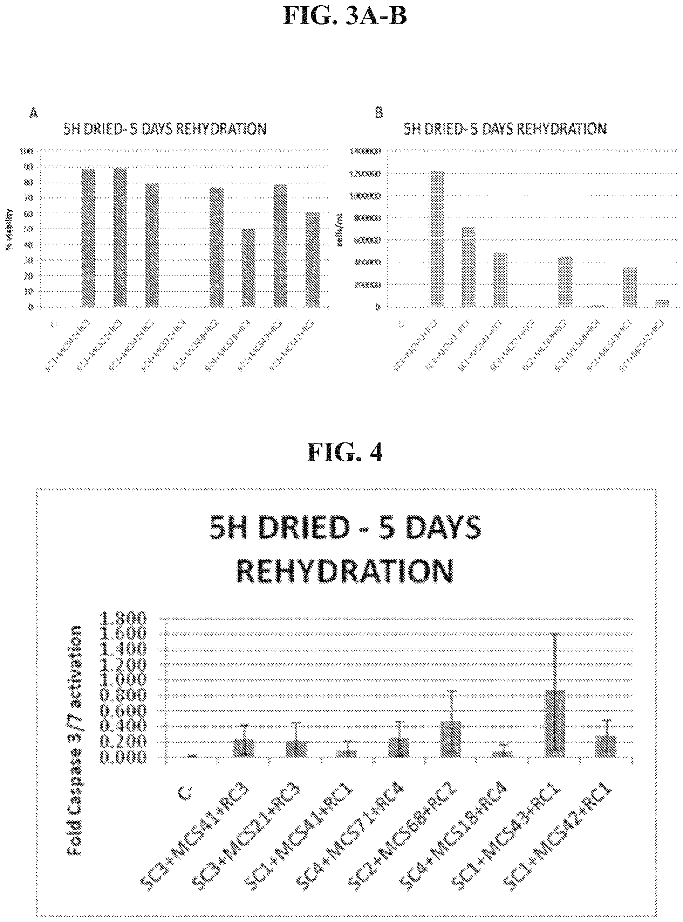

Briefly, HeLa cells were substantially dry stored using predetermined predehydration formulations (SC1, salubrinal and SC3, MLS-0315763) and a subset of the dehydration formulations set forth in Table 1 for a period of seven hours and then rehydrated using the rehydration formulations listed below (RC1, salubrinal and RC3, MLS-0315763). Cell viability was assessed seven days after rehydration using the Trypan Blue method. The results are shown in Table 3 and Figure 4.

**TABLE 3**

| EXEMPLARY FORMULATIONS MAINTAIN VIABLE CELLS AFTER SUBSTANTIALLY DRY STORAGE AFTER SEVEN DAYS POST-REHYDRATION | |
|---|---|
| **Formulations** | **% HeLa Cell Viability** |
| NF Control | 0 |
| Trehalose | 0 |
| SC1 + MCS41 + RC1 | 65 |
| SC1 + MCS42 + RC1 | 75 |
| SC1 + MCS43 + RC1 | 30 |
| SC3 + MCS41 + RC3 | 35 |

As shown in Table 3, after seven hour of rehydration unprotected control and trehalose stabilized cells did not yield in any viable cells whereas the exemplary formulations of the present invention maintained HeLa cell viability at various degrees for up to seven hours resulting in significant improvement over the gold standard trehalose dried cells.

### EXAMPLE 3

### FORMULATIONS COMPRISING EXEMPLARY APOPTOSIS INHIBITORS TARGETING THE ER STRESS PATHWAY MAINTAIN CELL VIABILITY DURING SUBSTANTIALLY DRY STORAGE FOR A PERIOD OF AT LEAST 120 HOURS

This Example demonstrates that a plurality of apoptosis inhibitors targeting different steps of the ER stress pathway when used in the formulations, compositions and methods described herein are capable of substantially dry storage of cells at ambient temperatures for a period of at least 24 hours.

Briefly, human neonatal fibroblasts were suspended in Cascade Media 106 and seeded as 100 ul cultures at a densities of 100-5000 cells per test in 96 well plates and incubated in an environment of ambient atmosphere while maintaining an elevated CO2 level (5%-10%) and temperature of 37C with relative humidity of 85-95%. The culture is adjusted to specific composition of predehydration formulations and mediaa specified concentration of each apoptosis inhibitor. The cells were incubated for a period of at least one hour but not more than 3 hours hr at 37°C. The predehydration formulation was thoroughly removed and 10-15 ul of MCS dehydration formulation 41 (Table 1) was added to each well. The cells were substantially air dried at 37C, 5% CO2, 20% relative humidity over a period of 90 minutes,stored at ambient temperature for a period of 24, 48 or 120 hours. At the appropriate time, the substantially dry stored cells were rehydrated by treating with a rehydration formulation comprising the same concentration of apoptosis inhibitor in 100-200 µl of predehydration formulation. Cell viability was determined by measuring ATP content as described herein.

The results are shown in Table 4:

**TABLE 4**

| FORMULATIONS COMPRISING EXEMPLARY APOPTOSIS INHIBITORS TARGETING THE ER STRESS PATHWAY MAINTAIN CELL VIABILITY DURING SUBSTANTIALLY DRY STORAGE FOR A PERIOD OF AT LEAST 120 HOURS | | | | | |
|---|---|---|---|---|---|
| | | | | | % CELL VIABILITY |
| **Pathway** | **Target** | **Inhibitor** | **24 hr** | **48 hr** | **120 hr** |
| NF Control | - | None | 0.0 | 0.0 | 0.0 |
| Cell | - | Trehalose | 7.5 | 0.0 | 0.0 |
| ER Stress | ASK-1 | MLS-0315763 | 103.4 | 76.1 | 55.9 |
| ER Stress | PI3K | LY294002 | 83.8 | 85.9 | 98.7 |
| ER Stress | GSK3b | CHIR98014 | 96.9 | 72.6 | 71.9 |
| ER Stress | ASK-1 | NDQI-1 | 99.2 | 100.0 | 76.9 |
| Autophagy Inducer | mTor | Rapamycin | 88.4 | 73.2 | 90.0 |
| ER Stress | NRF2 | L-sulphurophane | 98.8 | 65.3 | 50.9 |
| ER Stress | elf2-α | Salubrinal | 100.0 | 72.9 | 109.5 |
| Proliferation | MEK 1/2 | PD032591 | 100.0 | 85.1 | 95.7 |

As shown in Table 4, exemplary apoptosis inhibitors targeting various steps in the ER stress pathway maintain a substantial number of cells that are viable that retain at least one functional property 120 hours after dry storage. By 24 hr of dry storage, the untreated control and cells treated with the gold-standard trehalose maintained little to no viable cells whereas the formulations and methods described herein result in significant cell viability and the cells remain viable for a period of at least 120 hours of substantially dry storage.

### EXAMPLE 4

### SUBSTANTIALLY DRY STORED MESENCHYMAL STEM CELLS RETAIN THE ABILITY TO DIFFERENTIATE FOR A PERIOD OF AT LEAST TWO WEEKS POST-REHYDRATION

This Example demonstrates that mesenchymal stem cells (MSC) retain their ability to differentiate after substantially dry storage at ambient temperatures for a period of at least two weeks.

MSCs were substantially dry stored using 100nanomolar-2000 nanomoloar salubrinal and MSC Formulation 205 set forth in Table 1 and stored at room temperature for two weeks. The substantially dry stored cells were rehydrated in the presence of a rehydration formulation comprisingrehydration formulation containing salubrinal at similar concentrations, incubated for one hour under typical culture conditions (37C, 5% CO2, 85-95% RH) and which time the media was exchanged for MSC growth media and allowed to recover and proliferate for 48 hours and passaged into 12 well plates as monolayers (Fig 3 A-C) or as micromass cultures (Fig. 3D) before exposure to the StemPro™ Adipogenesis (B), Osteogenesis (C), and the Chondrogenesis (D) kit media preparations provided by LifeTechnologies™; cells were treated 21-30 days with feedings every 3 days. Clear changes in phenotypes relative to the undifferentiated MSC culture (A) are evident with apparent lipid globules consistent with adipocytes visible in (B), calciferous deposits consistent with osteocytes in (C), and column-like growth consistent with chondrocytes in (D) suggesting that the formulations described herein maintain the ability to and do not interfere with the differentiation potential of the MSCs.

Unless the context requires otherwise, throughout the present specification and claims, the word "comprise" and variations thereof, such as, "comprises" and "comprising" are to be construed in an open, inclusive sense, that is as "including, but not limited to".

Reference throughout this specification to "one embodiment" or "an embodiment" or "an aspect" means that a particular feature, structure or characteristic described in connection with the embodiment is included in at least one embodiment of the present invention. Thus, the appearances of the phrases "in one embodiment" or "in an embodiment" in various places throughout this specification are not necessarily all referring to the same embodiment. Furthermore, the particular features, structures, or characteristics may be combined in any suitable manner in one or more embodiments.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that methods and structures within the scope of these claims and their equivalents be covered thereby.

## Claims

1. A dehydration formulation, comprising:
(i) a pH buffer;
(ii) a synthetic amino acid;
(iii) a water-soluble polymer, and
(iv) a peptide, wherein the peptide is an alanine - glutamine dipeptide.

2. The dehydration formulation of claim 1, further comprising a non-reducing sugar, at least one apoptosis inhibitor, at least one apoptosis inhibitor and an ER chaperone inducer, at least one apoptosis inhibitor and an autophage inducer, a survival protein, or a second amino acid;
wherein the least one apopotosis inhibitor is selected from the group consisting of a PERK-eIF2-α inhibitor, an ASK1 inhibitor, a NRF2-KEAP1 inhibitor, a JNK inhibitor, a p38 MAP kinase inhibitor, an IRE1 inhibitor, a MEK inhibitor, a PI3K pathway inhibitor, a calpain inhibitor, a caspase-1 inhibitor, and combinations thereof; wherein
- the PERK-eIF2-α inhibitor is selected from the group consisting of salubrinal, Sal-003 (3-phenyl-N-[2,2,2-trichloro-1-[(4-chlorophenyl)carbamothioylamino]ethyl]prop-2-enamide), GSK 2606414 (7-Methyl-5-(1-{[3-(trifluoromethyl)phenyl]acetyl}-2,3-dihydro-1-H-indol-5-yl)7-H-pyrrolo[2,3d]pyrimidin-4-amine), GSK 2656157 (1-(5-(4-amino-7-methyl-7H-pyrrolo[2,3-d]pyrimidin-5-yl)-4-fluoroindolin-1-yl)-2-(6-methylpyridin-2-yl)ethanone) and ISRIB (trans-N,N'-(cyclohexane-1,4-diyl)bis(2-(4-chlorophenoxy)acetamide); or
- the ASK1 inhibitor is NDQI-1 or MLS-0315763; or
- the NRF2-KEAP1 inhibitor is selected from the group consisting of carnosic acid, tri-terpenoids, sulphoraphane, and tert-butylhydroquinone; or
- the MEK inhibitor is selected from the group consisting of PD0325901, N-[(2R)-2,3-dihydroxypropoxy]-3,4-difluoro-2-[(2-fluoro-4-iodophenyl)amino]-benzamide; MEK162 (5-[(4-bromo-2-fluorophenyl)amino]-4-fluoro-N-(2-hydroxyethoxy)-1-methyl-1H-benzimidazole-6-carboxamide), PD184352 (2-(2-chloro-4-iodophenylamino)-N-(cyclopropylmethoxy)-3,4-difluorobenzamide), pimasertib ((S)-N-(2,3-dihydroxypropyl)-3-(2-fluoro-4-iodophenylamino)isonicotinamide), selumetinib (6-(4-bromo-2-chlorophenylamino)-7-fluoro-N-(2-hydroxyethoxy)-3-methyl-3H-benzo[d]imidazole-5-carboxamide), trametinib (N-(3-(3-cyclopropyl-5-(2-fluoro-4-iodophenylamino)-6,8-dimethyl-2,4,7-trioxo-3,4,6,7-tetrahydropyrido[4,3-d]pyrimidin-1(2H)-yl)phenyl)acetamide), PD98059 (2-(2-amino-3-methoxyphenyl)-4H-chromen-4-one), and U0126-EtOH ((2Z,3Z)-2,3-bis(amino(2-aminophenylthio)methylene)succinonitrile,ethanol); or
- the PI3K inhibitor is selected from the group consisting of dactolisib (2-methyl-2-[4-(3-methyl-2-oxo-8-quinolin-3-ylimidazo[4,5-c]quinolin-1-yl)phenyl]propanenitrile), GDC-0941 (2-(1H-Indazol-4-yl)-6-[[4-(methylsulfonyl)-1-piperazinyl]methyl]-4-(4-morpholinyl)thieno[3,2-d]pyrimidine), LY294002 (2-(4-morpholinyl)-8-phenyl-4H-1-benzopyran-4-one), idealalisib (5-fluoro-3-phenyl-2-[(1S)-1-(7H-purin-6-ylamino)propyl]-4(3H)-quinazolinone), burparlisib (5-(2,6-dimorpholinopyrimidin-4-yl)-4-(trifluoromethyl)pyridin-2-amine), GDC-0032 (4-[5,6-dihydro-2-[3-methyl-1-(1-methylethyl)-1H-1,2,4-triazol-5-yl]imidazo[1,2-d][1,4]benzoxazepin-9-yl]-α,α-dimethyl-1H-pyrazole-1-acetamide), PI-103 (3-(4-(4-morpholinyl)pyrido[3',2':4,5]furo[3,2-d]pyrimidin-2-yl)phenol), NU7441 (8-(4-dibenzothienyl)-2-(4-morpholinyl)- 4H-1-benzopyran-4-one), GSK2636771 (2-methyl-1-[[2-methyl-3 -(trifluoromethyl)phenyl]methyl]-6-(4-morpholinyl)-1H-benzimidazole-4-carboxylic acid), IPI-145 (8-chloro-2-phenyl-3-[(1S)-1-(9-H-purin-6-ylamino)ethyl]-1-(2H)-isoquinolinone), XL147 (N-(3-(benzo[c][1,2,5]thiadiazol-5-ylamino)quinoxalin-2-yl)-4-methylbenzenesulfonamide), TGX-221 (7-methyl-2-(4-morpholinyl)-9-[1-(phenylamino)ethyl]-4H-pyrido[1,2-a]pyrimidin-4-one), PIK-90 (N-(7,8-Dimethoxy-2,3-dihydro-imidazo[1,2-c]quinazolin-5-yl)-nicotinamide), wortmannin (11-(acetyloxy)-1,6b,7,8,9a,10,11,11b-octahydro-1-(methoxymethyl)-9a,11b-dimethyl-, (1S,6bR,9aS,11R,11bR)-3H-fluoro[4,3,2-de]indeno[4,5-h]-2-benzopyran-3,6,9-trione), VS-5584 (5-[8-methyl-9-(1-methylethyl)-2-(4-morpholinyl)-9H-purin-6-yl]-2-pyrimidinamine), and TG-100703 (3-(2,4-diamino-6-pteridinyl)-phenol); or
- the IRE-1 inhibitor is selected from the group consisting of IRE1 Inhibitor I (N-[(2-hydroxynaphthalen-1-yl)methylidene]thiophene-2-sulfonamide), IRE1 Inhibitor II (3'-formyl-4'-hydroxy-5'-methoxybiphenyl-3-carboxamide), and IRE1 Inhibitor III (8-formyl-7-hydroxy-4-methylcoumarin, 7-hydroxy-4-methyl-2-oxo-2H-chromene-8-carbaldehyde);
- the calpain inhibitor is selected from the group consisting of Calpain Inhibitor I (N-Acetyl-Leu-Leu-Norleucine-CHO), Calpain Inhibitor II (N-Acetyl-Leu-Leu-Met), Calpain Inhibitor III (Z-Val-Phe-CHO), Calpain Inhibitor IV (Z-Leu-Leu-Tyr-CH₂F), Calpain Inhibitor V (Morpholinoureidyl-Val-homophenylalanine-CH₂F), Calpain Inhibitor VI (4-Fluorophenylsulfonyl-Val-Leu-CHO), Calpain Inhibitor X (Z-Leu-α-aminobutyric acid-CONHC₂H₅), Calpain Inhibitor XI (Z-L- α-aminobutyric acid-CONH(CH₂)₃-morpholine), and Calpain Inhibitor XII (Z-L-Norvaline-CONH-CH₂-2-Pyridyl);
- the caspase-1 inhibitor is selected from the group consisting of Caspase-1 Inhibitor II (Ac-YVAD-chloromethyl ketone), N-(2-Quinolyl)valyl-aspartyl-(2,6-difluorophenoxy)methyl ketone (in which the aspartyl residue is *O*-methylated or non-O-methylated), VX-765 ((S)-1-((S)-2-(4-amino-3-chlorobenzamido)-3,3-dimethylbutanoyl)-N-((2R,3S)-2-ethoxy-5-oxo-tetrahydrofuran-3-yl)pyrrolidine-2-carboxamide), and ZVAD-fluoromethyl ketone;
- the JNK inhibitor is selected from the group consisting of SP600125 (anthra[1-9-cd]pyrazol-6(2H)-one), JNK-IN-8 (3-[[4-(dimethylamino)-1-oxo-2-buten-1-yl]amino]-N-[3-methyl-4-[[4-(3-pyridinyl)-2-pyrimidinyl] amino]phenyl]-benzamide), and JNK-Inhibitor IX (N-(3-cyano-4,5,6,7-tetrahydrobenzo[b]thien-2-yl)- 1-naphthalenecarboxamide); and
- the p38 MAP kinase inhibitor is selected from the group consisting of SB203580 (4-(4-(4-fluorophenyl)-2-(4-(methylsulfinyl)phenyl)-1H-imidazol-5-yl)pyridine), LY2228820 (5-(2-tert-butyl-4-(4-fluorophenyl)-1H-imidazol-5-yl)-3-neopentyl-3H-imidazo[4,5-b]pyridin-2-amine dimethanesulfonate), PD169316 (4-(4-fluorophenyl)-2-(4-nitrophenyl)-5-(4-pyridyl)-1H-imidazole), PH-797804 (3-(4-(2,4-difluorobenzyloxy)-3-bromo-6-methyl-2-oxopyridin-1(2H)-yl)-N,4-dimethylbenzamide), SB202190 (4-(4-(4-fluorophenyl)-5-(pyridin-4-yl)-1H-imidazol-2-yl)phenol), BIRB 796 (Doramapimod; 1-(3-tert-butyl-1-p-tolyl-1H-pyrazol-5-yl)-3-(4-(2-morpholinoethoxy)naphthalen-1-yl)urea), VX-702 (1-(5-carbamoyl-6-(2,4-difluorophenyl)pyridin-2-yl)-1-(2,6-difluorophenyl)urea), and TAK-715 (N-[4-[2-ethyl-4-(3-methylphenyl)-5-thiazolyl]-2-pyridinyl]-benzamide;
wherein the ER chaperone inducer is selected from the group consisting of BIX, valproate, and lithium;
wherein the autophagy inducer is selected from the group consisting of fluspirilene, trifluoperazine, pimozide, nicardipine, niguldipine, loperamide, amiodarone, rapamycin, resveratrol, and SMERs;
wherein the survival protein is Bcl-xL.

3. The dehydration formulation of claim 1 or 2, wherein said dehydration formulation has a pH of about 4.0, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, 8.1, 8.2, 8.3, 8.4, 8.5, 8.6, 8.7, 8.8, 8.9 or 9.0, and wherein the term about is to be understood to represent quantitative variation that is more or less than the recited pH value by less than 0.25, preferably less than 0.1 pH unit.

4. The dehydration formulation of claim 3, wherein said dehydration formulation has a pH of about 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, and wherein the term about is to be understood to represent quantitative variation that is more or less than the recited pH value by less than 0.25, preferably less than 0.1 pH unit.

5. The dehydration formulation of any one of the preceding claims, wherein the water-soluble polymer is selected from polyvinyl pyrrolidine and polyvinyl alcohol.

6. The dehydration formulation of any one of claims 2-5, wherein the non-reducing sugar is sucrose or trehalose.

7. The dehydration formulation of any one of the preceding claims, wherein the pH buffer is selected from citric acid, tartaric acid, malic acid, sulfosalicylic acid, sulfoisophtalic acid, oxalic acid, borate, CAPS (3-(cyclohexylamino)-1-propanesulfonic acid), CAPSO (3-(cyclohexylamino)-2-hydroxy-1-propanesulfonic acid), EPPS (4-(2-hydroxyethyl)-1-piperazinepropanesulfonic acid), HEPES (4-(2-hydroxyethyl)piperazine-1-ethanesulfonic acid), MES (2-(N-morpholino)ethanesulfonic acid), MOPS (3-(N-morpholino)propanesulfonic acid), MOPSO (3-morpholino-2-hydroxypropanesulfonic acid), PIPES (1,4-piperazinediethanesulfonic acid), TAPS (N-[tris(hydroxymethyl)methyl]-3-aminopropanesulfonic acid), TAPSO (2-hydroxy-3-[tris(hydroxymethyl)methylamino]-1-propanesulfonic acid), TES (N-[tris(hydroxymethyl)methyl]-2-aminoethanesulfonic acid), bicine (N,N-Bis(2-hydroxyethyl)glycine), tricine (N-[Tris(hydroxymethyl)methyl]glycine), tris (tris(hydroxymethyl)aminomethane), and bis-tris (2-[Bis(2-hydroxyethyl)amino]-2-(hydroxymethyl)-1,3-propanediol).

8. The dehydration formulation of any one of the preceding claims, wherein the formulation further comprises a chelating agent selected from the group consisting of diethylenetriaminepentaacetic acid (DTPA), ethylenediaminetetraacetic acid (EDTA), ethylene glycol tetraacetic acid (EGTA), trans-1,2-diaminocyclohexane-N,N,N',N'-tetraacetic acid (CDTA), 1,2-bis(2-aminophenoxy)ethane-N,N,N',N'-tetraacetic acid (BAPTA), 1,4,7,10-tetraazacyclododecane-1,4,7,10-tetraacetic acid (DOTA), N-(2-hydroxyethyl)ethylenediamine-N,N',N'-triacetic acid, sodium gluconate, and nitrilotriacetic acid (NTA).

9. A method for substantially dry storage of one or more cell at ambient temperatures in the absence of refrigeration, comprising:
(i) incubating the one or more cell with a pre-dehydration formulation comprising at least one apoptosis inhibitor, wherein the least one apopotosis inhibitor is selected from the group consisting of a PERK-eIF2-α inhibitor, an ASK1 inhibitor, a NRF2-KEAP1 inhibitor, a JNK inhibitor, a p38 MAP kinase inhibitor, an IRE1 inhibitor, a GSK3 inhibitor, a MEK inhibitor, a PI3K pathway inhibitor, a calpain inhibitor, a caspase-1 inhibitor; wherein
- the PERK-eIF2-α inhibitor is selected from the group consisting of salubrinal, Sal-003 (3-phenyl-N-[2,2,2-trichloro-1-[(4-chlorophenyl)carbamothioylamino]ethyl]prop-2-enamide), GSK 2606414 (7-Methyl-5-(1-{[3-(trifluoromethyl)phenyl]acetyl}-2,3-dihydro-1-H-indol-5-yl)7-H-pyrrolo[2,3d]pyrimidin-4-amine), GSK 2656157 (1-(5-(4-amino-7-methyl-7H-pyrrolo[2,3-d]pyrimidin-5-yl)-4-fluoroindolin-1-yl)-2-(6-methylpyridin-2-yl)ethanone) and ISRIB (trans-N,N'-(cyclohexane-1,4-diyl)bis(2-(4-chlorophenoxy)acetamide); or
- the ASK1 inhibitor is NDQI-1 or MLS-0315763; or
- the NRF2-KEAP1 inhibitor is selected from the group consisting of carnosic acid, tri-terpenoids, sulphoraphane, and tert-butylhydroquinone; or
- the GSK3 inhibitor is selected from the group consisting of CHIR98014 (N6-[2-[[4-(2,4-dichlorophenyl)-5-(1-H-imidazol-2-yl)-2-pyrimidinyl]amino]ethyl]-3-nitro-2,6-pyridinediamine), valproate, CT 99021, and CT 20026; or
- the MEK inhibitor is selected from the group consisting of PD0325901, N-[(2R)-2,3-dihydroxypropoxy]-3,4-difluoro-2-[(2-fluoro-4-iodophenyl)amino]-benzamide; MEK162 (5-[(4-bromo-2-fluorophenyl)amino]-4-fluoro-N-(2-hydroxyethoxy)-1-methyl-1H-benzimidazole-6-carboxamide), PD184352 (2-(2-chloro-4-iodophenylamino)-N-(cyclopropylmethoxy)-3,4-difluorobenzamide), pimasertib ((S)-N-(2,3-dihydroxypropyl)-3-(2-fluoro-4-iodophenylamino)isonicotinamide), selumetinib (6-(4-bromo-2-chlorophenylamino)-7-fluoro-N-(2-hydroxyethoxy)-3-methyl-3H-benzo[d]imidazole-5-carboxamide), trametinib (N-(3-(3-cyclopropyl-5-(2-fluoro-4-iodophenylamino)-6,8-dimethyl-2,4,7-trioxo-3,4,6,7-tetrahydropyrido[4,3-d]pyrimidin-1(2H)-yl)phenyl)acetamide), PD98059 (2-(2-amino-3-methoxyphenyl)-4H-chromen-4-one), and U0126-EtOH ((2Z,3Z)-2,3-bis(amino(2 -aminophenylthio)methylene)succinonitrile,ethanol); or
- the PI3K inhibitor is selected from the group consisting of dactolisib (2-methyl-2-[4-(3-methyl-2-oxo-8-quinolin-3-ylimidazo[4,5-c]quinolin-1-yl)phenyl]propanenitrile), GDC-0941 (2-(1H-Indazol-4-yl)-6-[[4-(methylsulfonyl)-1-piperazinyl]methyl]-4-(4-morpholinyl)thieno[3,2-d]pyrimidine), LY294002 (2-(4-morpholinyl)-8-phenyl-4H-1-benzopyran-4-one), idealalisib (5-fluoro-3-phenyl-2-[(1*S*)-1-(7*H*-purin-6-ylamino)propyl]-4(3*H*)-quinazolinone), burparlisib (5-(2,6-dimorpholinopyrimidin-4-yl)-4-(trifluoromethyl)pyridin-2-amine), GDC-0032 (4-[5,6-dihydro-2-[3-methyl-1-(1-methylethyl)-1H-1,2,4-triazol-5-yl]imidazo[1,2-d][1,4]benzoxazepin-9-yl]-α,α-dimethyl-1H-pyrazole-1-acetamide), PI-103 (3-(4-(4-morpholinyl)pyrido[3',2':4,5]furo[3,2-d]pyrimidin-2-yl)phenol), NU7441 (8-(4-dibenzothienyl)-2-(4-morpholinyl)- 4H-1-benzopyran-4-one), GSK2636771 (2-methyl-1-[[2-methyl-3-(trifluoromethyl)phenyl]methyl]-6-(4-morpholinyl)-1H-benzimidazole-4-carboxylic acid), IPI-145 (8-chloro-2-phenyl-3-[(1S)-1-(9-H-purin-6-ylamino)ethyl]-1-(2H)-isoquinolinone), XL147 (N-(3-(benzo[c][1,2,5]thiadiazol-5-ylamino)quinoxalin-2-yl)-4-methylbenzenesulfonamide), TGX-221 (7-methyl-2-(4-morpholinyl)-9-[1-(phenylamino)ethyl]-4H-pyrido[1,2-a]pyrimidin-4-one), PIK-90 (N-(7,8-Dimethoxy-2,3-dihydro-imidazo[1,2-c]quinazolin-5-yl)-nicotinamide), wortmannin (11-(acetyloxy)-1,6b,7,8,9a,10,11,11b-octahydro-1-(methoxymethyl)-9a,11b-dimethyl-, (1S,6bR,9aS,11R,11bR)-3H-fluoro[4,3,2-de]indeno[4,5-h]-2-benzopyran-3,6,9-trione), VS-5584 (5-[8-methyl-9-(1-methylethyl)-2-(4-morpholinyl)-9H-purin-6-yl]- 2-pyrimidinamine), and TG-100703 (3-(2,4-diamino-6-pteridinyl)-phenol); or
- the IRE-1 inhibitor is selected from the group consisting of IRE1 Inhibitor I (N-[(2-hydroxynaphthalen-1-yl)methylidene]thiophene-2-sulfonamide), IRE1 Inhibitor II (3'-formyl-4'-hydroxy-5'-methoxybiphenyl-3-carboxamide), and IRE1 Inhibitor III (8-formyl-7-hydroxy-4-methylcoumarin, 7-hydroxy-4-methyl-2-oxo-2H-chromene-8-carbaldehyde);
- the calpain inhibitor is selected from the group consisting of Calpain Inhibitor I (N-Acetyl-Leu-Leu-Norleucine-CHO), Calpain Inhibitor II (N-Acetyl-Leu-Leu-Met), Calpain Inhibitor III (Z-Val-Phe-CHO), Calpain Inhibitor IV (Z-Leu-Leu-Tyr-CH2F), Calpain Inhibitor V (Morpholinoureidyl-Val-homophenylalanine-CH₂F), Calpain Inhibitor VI (4-Fluorophenylsulfonyl-Val-Leu-CHO), Calpain Inhibitor X (Z-Leu-α-aminobutyric acid-CONHC₂H₅), Calpain Inhibitor XI (Z-L-α-aminobutyric acid -CONH(CH₂)₃-morpholine), and Calpain Inhibitor XII (Z-L-Norvaline-CONH-CH₂-2-Pyridyl);
- the caspase-1 inhibitor is selected from the group consisting of Caspase-1 Inhibitor II (Ac-YVAD-chloromethyl ketone), N-(2-Quinolyl)valyl-aspartyl-(2,6-difluorophenoxy)methyl ketone (in which the aspartyl residue is *O*- methylated or non- O-methylated), VX-765 ((S)-1-((S)-2-(4-amino-3-chlorobenzamido)-3,3-dimethylbutanoyl)-N-((2R,3S)-2-ethoxy-5-oxo-tetrahydrofuran-3-yl)pyrrolidine-2-carboxamide), and ZVAD-fluoromethyl ketone;
- the JNK inhibitor is selected from the group consisting of SP600125 (anthra[1-9-cd]pyrazol-6(2H)-one), JNK-IN-8 (3-[[4-(dimethylamino)-1-oxo-2-buten-1-yl]amino]-N-[3-methyl-4-[[4-(3-pyridinyl)-2-pyrimidinyl]amino]phenyl]-benzamide), and JNK-Inhibitor IX (N-(3-cyano-4,5,6,7-tetrahydrobenzo[b]thien-2-yl)- 1-naphthalenecarboxamide); and
- the p38 MAP kinase inhibitor is selected from the group consisting of SB203580 (4-(4-(4-fluorophenyl)-2-(4-(methylsulfinyl)phenyl)-1H-imidazol-5-yl)pyridine), LY2228820 (5-(2-tert-butyl-4-(4-fluorophenyl)-1H-imidazol-5-yl)-3-neopentyl-3H-imidazo[4,5-b]pyridin-2-amine dimethanesulfonate), PD169316 (4-(4-fluorophenyl)-2-(4-nitrophenyl)-5-(4-pyridyl)-1H-imidazole), PH-797804 (3-(4-(2,4-difluorobenzyloxy)-3-bromo-6-methyl-2-oxopyridin-1(2H)-yl)-N,4-dimethylbenzamide), SB202190 (4-(4-(4-fluorophenyl)-5-(pyridin-4-yl)-1H-imidazol-2-yl)phenol), BIRB 796 (Doramapimod; 1-(3-tert-butyl-1-p-tolyl-1H-pyrazol-5-yl)-3-(4-(2-morpholinoethoxy)naphthalen-1-yl)urea), VX-702 (1-(5-carbamoyl-6-(2,4-difluorophenyl)pyridin-2-yl)-1-(2,6-difluorophenyl)urea), and TAK-715 (N-[4-[2-ethyl-4-(3-methylphenyl)-5-thiazolyl]-2-pyridinyl]-benzamide;
(ii) removing the pre-dehydration formulation from the one or more cell; and
(iii) dehydrating the one or more cell in the presence of the dehydration formulation as defined in any one of the claims 1-8.

10. The method of claim 9, wherein the apoptosis inhibitor is a reversible apoptosis inhibitor.

11. The method of claim 9, further comprising immobilizing one or more cell to a solid support prior to incubating the one or more cell with the pre-dehydration formulation.

12. The method of claim 9, further comprising rehydrating the one or more substantially dry stored cell to generate a rehydrated cell using a rehydration formulation comprising at least one apoptosis inhibitor.

13. The method of claim 9, wherein the at least one apoptosis inhibitor in the pre-dehydration formulation is the same as the at least one apoptosis inhibitor in the rehydration formulation.
